# EUROPEAN PATENT APPLICATION

(11) **EP 4 699 661 A2**
(43) Date of publication of application: **25.02.2026**
(21) Application number: 25214057.9
(22) Date of filing: 14.09.2022
(51) Int. Cl.: A61P 35/00

(54) **CLEAVABLE LINKERS**

(30) Priority: 14.09.2021 US 202163243843 P
(62) Divisional of application: 22801643.2
(71) Applicant: Xilio Development, Inc., Waltham, Massachusetts 02451 (US)
(72) Inventor: TOMAR, Dheeraj Singh, Bridgewater, Massachusetts, 02379 (US); PEDERZOLI-RIBEIL, Magali, Cambridge, Massachusetts, 02140 (US); KNUDSEN, Giselle Marcelline, San Anselmo, California, 94960 (US)
(74) Representative: CMS Cameron McKenna Nabarro Olswang LLP

(57) **Abstract**

The present invention discloses proteolytically cleavable peptide (CP) substrates, polypeptides, polypeptide constructs and prodrug constructs comprising the proteolytically cleavable peptides (e.g., cytokine prodrugs), and methods of use of the same.

## Description

### CROSS-REFERENCED APPLICATIONS

This application claims priority to, and the benefit of, U.S. provisional application No. 63/243,843, filed on September 14, 2021, the contents of which are hereby incorporated by reference in its entirety.

### SEQUENCE LISTING

The instant application contains a Sequence Listing which has been submitted electronically in XML format and is hereby incorporated by reference in its entirety. Said XML copy, created on September 8, 2022, is named XTX_P0112WO_SL.XML and is 14,003 bytes in size.

### BACKGROUND

Cleavable linkers are a conjugation tool for connecting components together, which can then be cleaved upon exposure to a reagent, to release said conjugated components. These types of linkers are a powerful tool used in chemical biology and the pharmaceutical field. In particular, cleavable linkers can be used in drugs and prodrugs for delivering therapeutic agents to a target of interest, for example to a tumor cell environment.

Cancer is the second leading cause of death in the United States, accounting for more deaths than the next five leading causes (chronic respiratory disease, stroke, accidents, Alzheimer's disease and diabetes). While great strides have been made especially with targeted therapies, there remains a great deal of work to do in this space. Immunotherapy and a branch of this field, immuno-oncology, is creating viable and exciting therapeutic options for treating malignancies. Specifically, it is now recognized that one hallmark of cancer is immune evasion and significant efforts have identified targets and developed therapies to these targets to reactivate the immune system to recognize and treat cancer.

Cytokine therapy is an effective strategy for stimulating the immune system to induce anti-tumor cytotoxicity. In particular, aldesleukin, a recombinant form of interleukin-2 (IL-2), has been approved by the FDA for the treatment of metastatic renal cell carcinoma and melanoma. Unfortunately, cytokines that are administered to patients generally have a very short half-life, thereby requiring frequent dosing. For instance, the product label of aldesleukin, marketed under the brand name Proleukin, states that the drug was shown to have a half-life of 85 minutes in patients who received a 5-minute intravenous (IV) infusion. In addition, administration of high doses of cytokine can cause adverse health outcomes, such as vascular leakage, through systemic immune activation. These findings illustrate the need for developing therapeutics, such as cytokine therapeutics, that effectively target tumors without the side effects associated with systemic immune activation.

Prodrugs in which a cytokine therapeutic is masked by a masking moiety and in which the therapeutic is only active after cleavage of the masking moiety in the tumor cell environment are one way envisaged for addressing this need.

### SUMMARY

This invention provides novel proteolytically cleavable peptide linkers comprising proteolytically cleavable peptides and their use in polypeptides. Said polypeptide may be an engineered polypeptide construct, comprising two components joined together via a proteolytically cleavable peptide linker of the invention.

The proteolytically cleavable peptide acts as a substrate for protease(s) (e.g., MMPs and/or cathepsin B). The proteolytically cleavable peptide linker is positioned within the polypeptide so that the linker cleaves by protease action in a protease rich environment, such as a tumor cell environment. Upon proteolytic cleavage, the polypeptide separates to form cleavage products. This invention also relates to cleavage products of said polypeptides, and methods related to the use of the same.

Provided herein is a polypeptide comprising a proteolytically cleavable peptide linker, wherein the linker comprises a proteolytically cleavable peptide (CP) consisting of the amino acid sequence PVSLRSGS (SEQ ID NO: 1), or GMPKDLYHAS (SEQ I DNO. 2), or RPLALWRS (SEQ ID NO: 3), or TQKPLGLS (SEQ ID NO:4), or APAGLIVPYN (SEQ ID NO:5), or PANLVAPDP (SEQ ID NO: 6), or IVGRPRHQGV (SEQ ID NO:7), or RSKYLATA(SEQ ID NO:8).

Also provided herein is a polypeptide construct comprising a proteolytically cleavable peptide linker, wherein the linker comprises a proteolytically cleavable peptide (CP) consisting of the amino acid sequence PVSLRSGS (SEQ ID NO: 1), or GMPKDLYHAS (SEQ ID NO: 2), or RPLALWRS (SEQ ID NO: 3), or TQKPLGLS (SEQ ID NO: 4), or APAGLIVPYN (SEQ ID NO:5), or PANLVAPDP (SEQ ID NO: 6), or IVGRPRHQGV(SEQ ID NO:7), or RSKYLATA(SEQ ID NO:8).

In some embodiments, the proteolytically cleavable peptide (CP) is PVSLRSGS (SEQ ID NO:1).

In some embodiments, the proteolytically cleavable peptide (CP) is GMPKDLYHAS (SEQ ID NO:2).

In some embodiments, the proteolytically cleavable peptide (CP) is RPLALWRS (SEQ ID NO:3).

In some embodiments, the proteolytically cleavable peptide (CP) is TQKPLGLS (SEQ ID NO:4).

In some embodiments, the proteolytically cleavable peptide (CP) is APAGLIVPYN (SEQ ID NO:5).

In some embodiments, the proteolytically cleavable peptide (CP) is PANLVAPDP (SEQ ID NO:6).

In some embodiments, the proteolytically cleavable peptide (CP) is IVGRPRHQGV (SEQ ID NO:7).

In some embodiments, the proteolytically cleavable peptide (CP) is RSKYLATA (SEQ ID NO:8).

In some embodiments, the proteolytically cleavable peptide linker is from 8 to 25 amino acids in length.

In some embodiments, the proteolytically cleavable peptide linker is from 10 to 25 amino acids in length.

In some embodiments, the proteolytically cleavable peptide linker is 8, 9, 10, 11, 12, 13, 14, 15, 16, 17, 18, 19, 20, 21, 22, 23, 24 or 25 amino acids in length.

In some embodiments, the proteolytically cleavable peptide linker comprises a motif PXXL (SEQ ID NO:11) (X can be any amino acid residue). As a nonlimiting example, the proteolytically cleavable peptide linker comprises a substrate sequence of PLGL (SEQ ID NO: 12). In some embodiments, the substrate sequence comprises PLAL (SEQ ID NO:13), PAGL (SEQ ID NO:14), PVSL (SEQ ID NO:15), or PANL (SEQ ID NO:16).

In some embodiments, the polypeptide construct comprises more than one proteolytically cleavable peptide linker.

In some embodiments, the polypeptide construct comprises a therapeutic moiety.

In some embodiments, the therapeutic moiety is a cytokine moiety.

In some embodiments, the proteolytically cleavable peptide linker is covalently bonded directly to the therapeutic moiety.

In some embodiments, the proteolytically cleavable peptide linker is located within the construct between the therapeutic moiety and a carrier moiety.

In some embodiments, the proteolytically cleavable peptide linker is located within the carrier moiety.

In some embodiments, the polypeptide construct comprises a single polypeptide chain.

In some embodiments, the polypeptide construct comprises more than one polypeptide chain.

In some embodiments, the proteolytically cleavable peptide linker is present in the same polypeptide chain as the therapeutic moiety.

In some embodiments, the proteolytically cleavable peptide linker is present in a different polypeptide chain to the therapeutic moiety.

In some embodiments, the polypeptide construct is a prodrug.

In some embodiments, the polypeptide or polypeptide construct further comprises a masking moiety.

In some embodiments, the masking moiety is present in the same polypeptide chain as the therapeutic moiety.

In some embodiments, the masking moiety is present in a different polypeptide chain to the therapeutic moiety.

In some embodiments, the polypeptide construct comprises a half-life extension moiety.

In some embodiments, the polynucleotide construct comprises a targeting moiety.

Further provided herein is a cytokine prodrug as described herein, wherein the cytokine prodrug comprises a masking moiety and the masking moiety comprises a domain of the extracellular domain of the cytokine receptor.

Also further provided herein is a masked cytokine comprising:
a first polypeptide chain comprising a masking moiety linked to a first half-life extension moiety via a first linker; and
a second polypeptide chain comprising a cytokine moiety thereof linked to a second half-life extension moiety via a second linker,
wherein the first half-life extension moiety is associated with the second half-life extension moiety, and
wherein at least the first linker or the second linker is a proteolytically cleavable peptide linker comprising a proteolytically cleavable peptide (CP) consisting of the amino acid sequence PVSLRSGS (SEQ ID NO:1), or GMPKDLYHAS (SEQ ID NO:2), or RPLALWRS (SEQ ID NO:3), or TQKPLGLS (SEQ ID NO: 4), or APAGLIVPYN (SEQ ID NO:5), or PANLVAPDP (SEQ ID NO:6), or IVGRPRHQGV (SEQ ID NO:7), or RSKYLATA (SEQ ID NO:8).

In some embodiments, the first proteolytically cleavable peptide linker and the second proteolytically cleavable peptide linker comprise the same amino acid sequence. In other embodiments, the first proteolytically cleavable peptide linker and the second proteolytically cleavable peptide linker comprise different amino acid sequences.

In some embodiments, the first polypeptide chain comprises:
N' HL1-L1-MM C'
and the second polypeptide chain comprises:
   N' HL2-L2-C C'
where HL1 is the first half life extension domain, L1 is the first linker, MM is the masking moiety, HL2 is the second half life extension domain, L2 is the second linker, and C is the cytokine moiety,
wherein the first half-life extension moiety is associated with the second half-life extension moiety, and
wherein at least the first linker or the second linker is a proteolytically cleavable peptide linker comprising a proteolytically cleavable peptide (CP) consisting of the amino acid sequence PVSLRSGS (SEQ ID NO: 1) or GMPKDLYHAS (SEQ ID NO:2), or RPLALWRS (SEQ ID NO:3), or TQKPLGLS (SEQ ID NO:4), or APAGLIVPYN (SEQ ID NO:5), or PANLVAPDP (SEQ ID NO: 6), or IVGRPRHQGV (SEQ ID NO: 7), or RSKYLATA (SEQ ID NO:8).

Also provided herein is a masked cytokine comprising a polypeptide chain comprising formula:

**N' HL-L2-C-L1-MM** C'

where HL is the half-life extension domain, L1 is the first linker, MM is the masking moiety, L2 is the second linker, and C is the cytokine moiety, wherein at least the first linker comprises a proteolytically cleavable peptide linker comprising a proteolytically cleavable peptide (CP) consisting of the amino acid sequence PVSLRSGS (SEQ ID NO: 1) or GMPKDLYHAS (SEQ ID NO:2) , or RPLALWRS (SEQ ID NO:3), or TQKPLGLS (SEQ ID NO:4), or APAGLIVPYN (SEQ ID NO:5), or PANLVAPDP (SEQ ID NO:6), or IVGRPRHQGV (SEQ ID NO:7), or RSKYLATA (SEQ ID NO:8).

Also provided herein is a masked cytokine comprising a polypeptide chain comprising formula:

**N' HL-L2-MM-L1-C** C'

where HL is the half-life extension domain, L1 is the first linker, MM is the masking moiety, L2 is the second linker, and C is the cytokine moiety thereof, wherein at least the first linker comprises a proteolytically cleavable peptide linker comprising a proteolytically cleavable peptide (CP) consisting of the amino acid sequence PVSLRSGS (SEQ ID NO:1) or GMPKDLYHAS (SEQ ID NO:2), or RPLALWRS (SEQ ID NO:3), or TQKPLGLS (SEQ ID NO:4), or APAGLIVPYN (SEQ ID NO:5), or PANLVAPDP (SEQ ID NO:6), or IVGRPRHQGV (SEQ ID NO:7), or RSKYLATA (SEQ ID NO:8). Also provided herein is a targeted cytokine comprising: a targeting moiety; a cytokine or a fragment thereof; a masking moiety; and a half-life extension domain comprising a first Fc polypeptide linked to the cytokine or a fragment thereof through a first linker and a second Fc polypeptide linked to the masking moiety through a second linker, and wherein the targeting moiety is linked to the Fc domain through one or both of the first and second Fc polypeptides. In this targeted cytokine construct, the first or the second linker is a cleavable linker such that the masking moiety releases the cytokine or a fragment thereof upon cleavage. The first cleavable linker or the second cleavable linker comprises a proteolytically cleavable peptide linker comprising a proteolytically cleavable peptide (CP) consisting of the amino acid sequence PVSLRSGS (SEQ ID NO: 1) or GMPKDLYHAS (SEQ ID NO:2), or RPLALWRS (SEQ ID NO:3), or TQKPLGLS (SEQ ID NO:4), or APAGLIVPYN (SEQ ID NO:5), or PANLVAPDP (SEQ ID NO:6), or IVGRPRHQGV (SEQ ID NO:7), or RSKYLATA (SEQ ID NO:8).

In some embodiments, the second linker is the proteolytically cleavable linker and the first linker is a non-cleavable linker.

In some embodiments, the first linker is the proteolytically cleavable linker and the second linker is a non-cleavable linker.

In some embodiments, the proteolytically cleavable peptide linker is from 9 to 25 amino acids in length.

In some embodiments, the proteolytically cleavable peptide (CP) consists of the amino acid sequence PVSLRSGS (SEQ ID NO: 1).

In some embodiments, the proteolytically cleavable peptide (CP) consists of the amino acid sequence GMPKDLYHAS (SEQ ID NO:2).

In some embodiments, the proteolytically cleavable peptide (CP) consists of the amino acid sequence of RPLALWRS (SEQ ID NO:3).

In some embodiments, the proteolytically cleavable peptide (CP) consists of the amino acid sequence of TQKPLGLS (SEQ ID NO:4).

In some embodiments, the proteolytically cleavable peptide (CP) consists of the amino acid sequence of APAGLIVPYN (SEQ ID NO:5).

In some embodiments, the proteolytically cleavable peptide (CP) consists of the amino acid sequence of PANLVAPDP (SEQ ID NO:6).

In some embodiments, the proteolytically cleavable peptide (CP) consists of the amino acid sequence of IVGRPRHQGV (SEQ ID NO:7).

In some embodiments, the proteolytically cleavable peptide (CP) consists of the amino acid sequence of RSKYLATA (SEQ ID NO:8).

In some embodiments, the cytokine moiety comprises a wild-type cytokine moiety or variant cytokine moiety.

In some embodiments, the cytokine moiety is an IL-2 cytokine moiety.

In some embodiments, the IL-2 cytokine moiety comprises an IL-2 cytokine or fragment thereof.

In some embodiments, the cytokine moiety is an IL-12 cytokine moiety.

In some embodiments, the IL-12 cytokine moiety comprises an IL-12 cytokine or fragment thereof.

In some embodiments, the cytokine moiety is an IL-15 cytokine moiety.

In some embodiments, the IL-15 cytokine moiety comprises an IL-15 cytokine or fragment thereof.

In some embodiments, the masked cytokine further comprises a domain comprising an IL-15Rα subunit or a functional fragment thereof ('IL-15Rα domain').

In some embodiments, the masking moiety comprises a domain of the extracellular domain of the cytokine receptor.

In some embodiments, the half-life extension domains are each an Fc domain or a fragment thereof.

In some embodiments, the targeting moiety is an antibody or antigen binding fragment thereof.

Provided herein is a cleavage product preparable by proteolytic cleavage of the proteolytically cleavable linker in a polypeptide or polypeptide construct, a cytokine prodrug, a masked cytokine, or a targeted cytokine as described anywhere herein.

Provided herein is a nucleic acid encoding a polypeptide construct as described anywhere herein.

Provided herein is a nucleic acid encoding a cytokine prodrug as described anywhere herein.

Provided herein is a nucleic acid encoding a masked cytokine as described anywhere herein.

Provided herein is a nucleic acid encoding a targeted cytokine as described anywhere herein.

Provided herein is a nucleic acid encoding a cleavage product as described anywhere herein.

Provided herein is a vector comprising a nucleic acid as described anywhere herein.

Provided herein is a host cell comprising a nucleic acid as described anywhere herein.

Provided herein is a host cell as described anywhere herein, wherein the host cell is a HEK cell. In some embodiments, the host cell is a CHO cell.

Provided herein is a composition comprising the polypeptide construct as described anywhere herein.

Provided herein is a composition comprising a cytokine prodrug as described anywhere herein.

Provided herein is a composition comprising a masked cytokine as described anywhere herein.

Provided herein is a composition comprising a targeted cytokine as described anywhere herein.

Provided herein is a pharmaceutical composition comprising a polypeptide construct as described anywhere herein and a pharmaceutically acceptable carrier.

Provided herein is a pharmaceutical composition comprising a cytokine prodrug as described anywhere herein, and a pharmaceutically acceptable carrier.

Provided herein is a pharmaceutical composition comprising a masked cytokine as described anywhere herein, and a pharmaceutically acceptable carrier.

Provided herein is a pharmaceutical composition comprising a targeted cytokine as described anywhere herein, and a pharmaceutically acceptable carrier.

Provided herein is a kit comprising a polypeptide construct, or a cytokine prodrug, or a masked cytokine, or a targeted cytokine, or the composition, or the pharmaceutical composition, as described anywhere herein.

Provided herein is a method of producing a masked cytokine as described anywhere herein, comprising culturing the host cell as described herein under a condition that produces the masked cytokine.

Provided herein is a method of producing a targeted cytokine as described anywhere herein, comprising culturing the host cell as described herein under a condition that produces the masked cytokine.

Provided herein is a composition comprising a cleavage product as described anywhere herein.

Provided herein is a pharmaceutical composition comprising the cleavage product as described anywhere herein, and a pharmaceutically acceptable carrier.

Provided herein is a polypeptide construct as described anywhere herein for use in medicine.

Provided herein is a cytokine prodrug as described anywhere herein for use in medicine.

Provided herein is a masked cytokine as described anywhere herein for use in medicine.

Provided herein is a targeted cytokine as described anywhere herein for use in medicine.

Provided herein is a composition as described anywhere herein for use in medicine.

Provided herein is a pharmaceutical composition as described anywhere herein for use in medicine.

Provided herein is a cleavage product as described anywhere herein for use in medicine.

Provided herein is a method of treating or preventing cancer in a subject, the method comprising administering to the subject an effective amount of a polypeptide construct as described anywhere herein.

Provided herein is a method of treating or preventing cancer in a subject, the method comprising administering to the subject an effective amount of a cytokine prodrug as described anywhere herein.

Provided herein is a method of treating or preventing cancer in a subject, the method comprising administering to the subject an effective amount of a masked cytokine as described anywhere herein.

Provided herein is a method of treating or preventing cancer in a subject, the method comprising administering to the subject an effective amount of a targeted cytokine as described anywhere herein.

Provided herein is a method of treating or preventing cancer in a subject, the method comprising administering to the subject an effective amount of a composition as described anywhere herein.

Provided herein is a method of treating or preventing cancer in a subject, the method comprising administering to the subject an effective amount of a pharmaceutical composition as described anywhere herein.

Provided herein is a method of treating or preventing cancer in a subject, the method comprising administering to the subject an effective amount of a cleavage product as described anywhere herein.

Provided herein is a method of treating or preventing cancer in a subject, the method comprising administering to the subject an effective amount of a cytokine prodrug, or a masked cytokine or a targeted cytokine as described anywhere herein, whereby the cytokine prodrug or masked cytokine, or targeted cytokine is proteolytically cleaved in vivo to produce a cleavage product as described anywhere herein.

Provided herein is a method of treating or preventing cancer in a subject, the method comprising a step of producing a cleavage product in vivo that is capable of binding to its cognate receptor, where the cleavage product is as described anywhere herein.

In some embodiments, the cancer is a solid tumor.

Provided herein is a polypeptide construct as described anywhere herein for use in treating or preventing cancer.

Provided herein is a cytokine prodrug as described anywhere herein for use in treating or preventing cancer.

Provided herein is a masked cytokine as described anywhere herein for use in treating or preventing cancer.

Provided herein is a targeted cytokine as described anywhere herein for use in treating or preventing cancer.

Provided herein is a composition as described anywhere herein for use in treating or preventing cancer.

Provided herein is a pharmaceutical composition as described anywhere herein for use in treating or preventing cancer.

Provided herein is a cleavage product as described anywhere herein for use in treating or preventing cancer.

Provided herein is a cleavage product as described anywhere herein for use in a method of treating or preventing cancer, the method comprising a step of a polypeptide drug construct, a cytokine prodrug, or a masked cytokine, or a targeted cytokine as described anywhere herein to a patient, thereby producing the cleavage product by proteolytic cleavage of the proteolytically cleavable peptide linker in vivo.

Provided herein is a cleavage product as described anywhere herein for use in a method of treating or preventing cancer in a subject, the method comprising a step of producing the cleavage product by in vivo proteolytic cleavage of a polypeptide drug construct, a cytokine prodrug, or a masked cytokine, or a targeted cytokine as described anywhere herein, that has been administered to the subject.

### BRIEF DESCRIPTION OF THE DRAWINGS

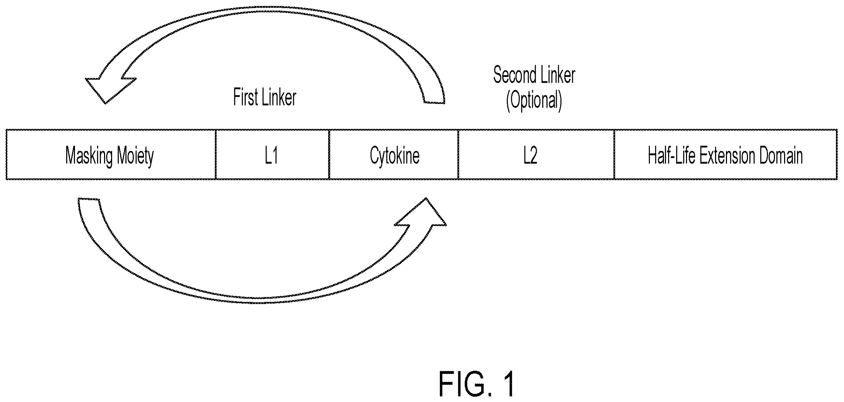

FIG. 1 shows the structure of exemplary embodiments of a masked cytokine that includes a masking moiety, a cytokine or functional fragment thereof ("cytokine"), a half-life extension moiety, and a first linker. These exemplary embodiments also include a second linker. The first or second linker may be a cleavable linker. As shown by the arrows, while the exemplary embodiments shows the masking moiety linked to the first linker, and the cytokine or functional fragment thereof is linked to the first linker and the second linker, the masking moiety and the cytokine or functional fragment thereof can be interchanged such that the cytokine or functional fragment thereof is linked to the first linker, and the masking moiety is linked to the first linker and the second linker. FIG. 1 shows the structure of an exemplary embodiment of a masked cytokine as a linear monomer.

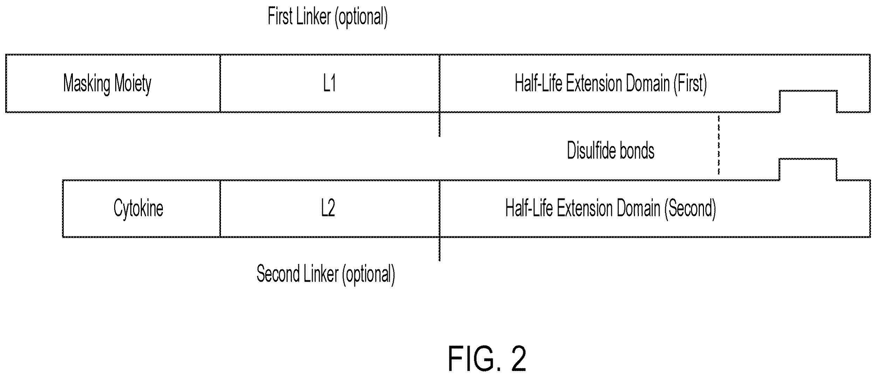

FIG. 2 shows the structure of an exemplary embodiment of a masked cytokine that includes a masking moiety, a cytokine or functional fragment thereof ("cytokine"), a first half-life extension moiety, and a second half-life extension moiety. The exemplary embodiment shown in FIG. 2 also includes a first linker, and a second linker. The first or second linker may be a cleavable linker. The exemplary first and second half-life extension moieties include "knobs into holes" modifications that promote the association of the first half-life extension moiety with the second half-life extension moiety, as shown by the "hole" in the first half-life extension moiety and the "knob" in the second half-life extension moiety. The first half-life extension moiety and the second half-life extension moiety are also shown as associating, at least in part, due to the formation of disulfide bonds. It is to be understood that although the "hole" is depicted as part of the first half-life extension moiety (linked to the masking moiety) and the "knob" is depicted as part of the second half-life extension moiety (linked to the cytokine), the "hole" and the "knob" can alternatively be included in the second half-life extension moiety and the first half-life extension moiety, respectively, so that the "hole" is a part of the second half-life extension moiety (linked to the cytokine) and the "knob" is part of the first half-life extension moiety (linked to masking moiety).
FIG. 3A is a representative diagram of the "heterodimeric" masked cytokine (Structure A) wherein the proteolytically cleavable peptide linker is positioned between the half-life extension moiety and the masking moiety.
FIG. 3B is a representative diagram of the "heterodimeric" masked cytokine (Structure B) wherein the proteolytically cleavable peptide linker is positioned between the half-life extension moiety and the cytokine moiety.
FIG. 3C is a representative diagram of a "heterodimeric" targeted cytokine (Structure C), wherein an antibody is linked to a cytokine moiety and a masking moiety. The proteolytically cleavable peptide linker is positioned on the masking moiety arm.
FIG. 3D is a representative diagram of a "heterodimeric" targeted masked cytokine (Structure D), wherein an antibody is linked to a cytokine moiety and a masking moiety. The proteolytically cleavable peptide linker is positioned on the masking moiety arm.

### DETAILED DESCRIPTION

### 1. POLYPEPTIDE CONSTRUCTS

This invention provides novel proteolytically cleavable peptide linkers.

The proteolytically cleavable peptide linker is positioned within a polypeptide, in particular a polypeptide construct so that when the linker cleaves by protease action, the polypeptide construct separates. Thus, the invention relates to a polypeptide construct comprising a proteolytically cleavable peptide linker as described anywhere herein.

This invention also relates to cleavage products of said polypeptide constructs.

Protease substrate amino acid sequences PVSLRSGS (SEQ ID NO:1) or GMPKDLYHAS (SEQ ID NO:2), or RPLALWRS (SEQ ID NO:3), or TQKPLGLS (SEQ ID NO:4), or APAGLIVPYN (SEQ ID NO:5), or PANLVAPDP (SEQ ID NO:6), or IVGRPRHQGV (SEQ ID NO:7), or RSKYLATA (SEQ ID NO:8) have been found to demonstrate very specific cleavage. Thus, these proteolytically cleavable peptides (CPs) advantageously can be used in the proteolytically cleavable peptide linkers of the invention. In particular, these proteolytically cleavable peptides have been found to demonstrate very specific cleavage in the tumor cell environment compared to non-tumor cell environment. Thus, these proteolytically cleavable peptides advantageously demonstrate tumor-specific cleavage.

A polypeptide construct of the invention may comprise any number and/or type of component. The proteolytically cleavable peptide linkers of the invention may be used to conjugate together said components in a polypeptide construct. In some embodiments, the proteolytically cleavable peptide linker may be used to conjugate one group of components to another group of components. In some embodiments, said group of components comprises 1, 2, 3, 4, or 5 or more components.

In some embodiments, it may be advantageous for certain components in the polypeptide construct to separate and for other components to remain linked. For example, in some embodiments, the proteolytically cleavable peptide linker may be used to conjugate a targeting moiety to a therapeutic moiety, where it is desirable for the therapeutic moiety to separate from the targeting moiety, such as once the polypeptide construct has reached a target environment.

### 2. PROTEOLYTICALLY CLEAVABLE PEPTIDE LINKERS

The proteolytically cleavable peptide linkers described herein comprise a proteolytically cleavable peptide (CP) consisting of the amino acid sequence PVSLRSGS (SEQ ID NO: 1) or GMPKDLYHAS (SEQ ID NO:2) , or RPLALWRS 9 SEQ ID NO:3), or TQKPLGLS (SEQ ID NO:4), or APAGLIVPYN (SEQ ID NO:5), or PANLVAPDP (SEQ ID NO:6), or IVGRPRHQGV (SEQ ID NO:7) , or RSKYLATA (SEQ ID NO:8).

A first aspect of the invention relates to the protease substrate amino acid sequence PVSLRSGS (SEQ ID NO:1).

In one embodiment of the first aspect, the proteolytically cleavable peptide (CP) consists of the amino acid sequence PVSLRSGS (SEQ ID NO: 1).

In another embodiment of the first aspect, the proteolytically cleavable peptide linker comprises a proteolytically cleavable peptide (CP) consisting of the amino acid sequence PVSLRSGS (SEQ ID NO:1), and is from 8 to 25, 9 to 25, 10 to 25, 10 to 20, 12 to 20, or 12 to 18 amino acids in length. In yet another embodiment of the first aspect, the proteolytically cleavable peptide linker comprises a proteolytically cleavable peptide (CP) consisting of the sequence of PVSLRSGS (SEQ ID NO:1) with less than 4, or less than 3, or less than 2 amino acid substitutions.

A second aspect of the invention relates to the protease substrate amino acid sequence GMPKDLYHAS (SEQ ID NO:2).

In one embodiment of the second aspect, the proteolytically cleavable peptide (CP) consists of the amino acid sequence GMPKDLYHAS (SEQ ID NO:2).

In another embodiment of the second aspect, the proteolytically cleavable peptide linker comprises a proteolytically cleavable peptide (CP) consisting of the amino acid sequence GMPKDLYHAS (SEQ ID NO:2), and is from 10 to 25, 11 to 25, 12 to 25, 10 to 20, 12 to 20, or 12 to 18 amino acids in length. In yet another embodiment of the second aspect, the proteolytically cleavable peptide linker comprises a proteolytically cleavable peptide (CP) consisting of the sequence of GMPKDLYHAS (SEQ ID NO:2) with less than 5, or less than 4, or less than 3, or less than 2 amino acid substitutions.

A third aspect of the invention relates to the protease substrate amino acid sequence RPLALWRS (SEQ ID NO:3).

In one embodiment of the third aspect, the proteolytically cleavable peptide (CP) consists of the amino acid sequence RPLALWRS (SEQ ID NO:3).

In another embodiment of the third aspect, the proteolytically cleavable peptide linker comprises a proteolytically cleavable peptide (CP) consisting of the amino acid sequence RPLALWRS (SEQ ID NO:3), and is from 8 to 25, 9 to 25, 10 to 25, 10 to 20, 12 to 20, or 12 to 18 amino acids in length. In yet another embodiment of the third aspect, the proteolytically cleavable peptide linker comprises a proteolytically cleavable peptide (CP) consisting of the sequence of RPLALWRS (SEQ ID NO:3) with less than 4, or less than 3, or less than 2 amino acid substitutions.

A fourth aspect of the invention relates to the protease substrate amino acid sequence TQKPLGLS (SEQ ID NO:4).

In one embodiment of the fourth aspect, the proteolytically cleavable peptide (CP) consists of the amino acid sequence TQKPLGLS (SEQ ID NO:4).

In another embodiment of the fourth aspect, the proteolytically cleavable peptide linker comprises a proteolytically cleavable peptide (CP) consisting of the amino acid sequence TQKPLGLS (SEQ ID NO:4), and is from 8 to 25, 9 to 25, 10 to 25, 10 to 20, 12 to 20, or 12 to 18 amino acids in length. In yet another embodiment of the fourth aspect, the proteolytically cleavable peptide linker comprises a proteolytically cleavable peptide (CP) consisting of the sequence of TQKPLGLS (SEQ ID NO:4), with less than 4, or less than 3, or less than 2 amino acid substitutions.

A fifth aspect of the invention relates to the protease substrate amino acid sequence APAGLIVPYN (SEQ ID NO:5).

In one embodiment of the fifth aspect, the proteolytically cleavable peptide (CP) consists of the amino acid sequence APAGLIVPYN (SEQ ID NO:5).

In another embodiment of the fifth aspect, the proteolytically cleavable peptide linker comprises a proteolytically cleavable peptide (CP) consisting of the amino acid sequence APAGLIVPYN (SEQ ID NO:5), and is from 10 to 25, 11 to 25, 12 to 25, 10 to 20, 12 to 20, or 12 to 18 amino acids in length. In yet another embodiment of the fifth aspect, the proteolytically cleavable peptide linker comprises a proteolytically cleavable peptide (CP) consisting of the sequence of APAGLIVPYN (SEQ ID NO:5) with less than 5, less than 4, or less than 3, or less than 2 amino acid substitutions.

A sixth aspect of the invention relates to the protease substrate amino acid sequence PANLVAPDP (SEQ ID NO:6).

In one embodiment of the sixth aspect, the proteolytically cleavable peptide (CP) consists of the amino acid sequence PANLVAPDP (SEQ ID NO:6).

In another embodiment of the sixth aspect, the proteolytically cleavable peptide linker comprises a proteolytically cleavable peptide (CP) consisting of the amino acid sequence PANLVAPDP (SEQ ID NO:6), and is from 9 to 25, 10 to 25, 11 to 25, 12 to 25, 10 to 20, 12 to 20, or 12 to 18 amino acids in length. In yet another embodiment of the sixth aspect, the proteolytically cleavable peptide linker comprises a proteolytically cleavable peptide (CP) consisting of the sequence of PANLVAPDP (SEQ ID NO:6), with less than 4, or less than 3, or less than 2 amino acid substitutions.

A seventh aspect of the invention relates to the protease substrate amino acid sequence IVGRPRHQGV (SEQ ID NO:7).

In one embodiment of the seventh aspect, the proteolytically cleavable peptide (CP) consists of the amino acid sequence IVGRPRHQGV (SEQ ID NO:7).

In another embodiment of the seventh aspect, the proteolytically cleavable peptide linker comprises a proteolytically cleavable peptide (CP) consisting of the amino acid sequence IVGRPRHQGV (SEQ ID NO:7), and is from 10 to 25, 11 to 25, 12 to 25, 10 to 20, 12 to 20, or 12 to 18 amino acids in length. In yet another embodiment of the seventh aspect, the proteolytically cleavable peptide linker comprises a proteolytically cleavable peptide (CP) consisting of the sequence of IVGRPRHQGV (SEQ ID NO:7), with less than 5, or less than 4, or less than 3, or less than 2 amino acid substitutions.

An eighth aspect of the invention relates to the protease substrate amino acid RSKYLATA.

In one embodiment of the eighth aspect, the proteolytically cleavable peptide (CP) consists of the amino acid sequence RSKYLATA (SEQ ID NO:8).

In another embodiment of the eighth aspect, the proteolytically cleavable peptide linker comprises a proteolytically cleavable peptide (CP) consisting of the amino acid sequence RSKYLATA (SEQ ID NO:8), and is from 8 to 25, 9 to 25, 10 to 25, 11 to 25, 12 to 25, 10 to 20, 12 to 20, or 12 to 18 amino acids in length. In yet another embodiment of the eighth aspect, the proteolytically cleavable peptide linker comprises a proteolytically cleavable peptide (CP) consisting of the sequence of RSKYLATA (SEQ ID NO:8), with less than 4, or less than 3, or less than 2 amino acid substitutions.

In some embodiments, the proteolytically cleavable peptide linker is 8, 9, 10, 11, 12, 13, 14, 15, 16, 17, 18, 19, 20, 21, 22, 23, 24 or 25 amino acids in length.

In some embodiments, the proteolytically cleavable peptide linker comprises a motif PXXL (SEQ ID NO:11) (X can be any amino acid residue). As a nonlimiting example, the proteolytically cleavable peptide linker comprises a substrate sequence of PLGL (SEQ ID NO: 12). In some embodiments, the substrate sequence comprises PLAL (SEQ ID NO:13), PAGL (SEQ ID NO:14), PVSL (SEQ ID NO:15), or PANL (SEQ ID NO:16).

Any polypeptide or polypeptide construct described herein may comprise a proteolytically cleavable peptide (CP) or proteolytically cleavable linker according to the first aspect of the invention.

Any polypeptide or polypeptide construct described herein may comprise a proteolytically cleavable peptide (CP) or proteolytically cleavable linker according to the second aspect of the invention.

Any polypeptide or polypeptide construct described herein may comprise a proteolytically cleavable peptide (CP) or proteolytically cleavable linker according to the third aspect of the invention.

Any polypeptide or polypeptide construct described herein may comprise a proteolytically cleavable peptide (CP) or proteolytically cleavable linker according to the fourth aspect of the invention.

Any polypeptide or polypeptide construct described herein may comprise a proteolytically cleavable peptide (CP) or proteolytically cleavable linker according to the fifth aspect of the invention.

Any polypeptide or polypeptide construct described herein may comprise a proteolytically cleavable peptide (CP) or proteolytically cleavable linker according to the sixth aspect of the invention.

Any polypeptide or polypeptide construct described herein may comprise a proteolytically cleavable peptide (CP) or proteolytically cleavable linker according to the seventh aspect of the invention.

Any polypeptide or polypeptide construct described herein may comprise a proteolytically cleavable peptide (CP) or proteolytically cleavable linker according to the eighth aspect of the invention.

### 3. POLYPEPTIDE DRUG CONSTRUCTS

The invention also relates to the use of the proteolytically cleavable peptide linkers as described anywhere herein in polypeptide drug constructs for delivering a therapeutic moiety to a target of interest, and methods related to the use of the same. Accordingly, provided herein also include polypeptide drug constructs comprising the proteolytically cleavable peptide linkers as described herein.

Due to their tumor specificity, cleavable peptides PVSLRSGS (SEQ ID NO:1) or GMPKDLYHAS (SEQ ID NO:2), or RPLALWRS (SEQ ID NO:3), or TQKPLGLS (SEQ ID NO:4), or APAGLIVPYN (SEQ ID NO:5), or PANLVAPDP (SEQ ID NO:6), or IVGRPRHQGV (SEQ ID NO:7), or RSKYLATA (SEQ ID NO:8) advantageously can be used in proteolytically cleavable peptide linkers in polypeptide drug constructs where the target of the drug is a tumor cell, wherein any systemic side effects of the administered protein therapeutic may be reduced. In some embodiments, cleavable peptides PVSLRSGS (SEQ ID NO: 1) or GMPKDLYHAS (SEQ ID NO:2), or RPLALWRS (SEQ ID NO:3), or TQKPLGLS (SEQ ID NO:4), or APAGLIVPYN (SEQ ID NO:5), or PANLVAPDP (SEQ ID NO:6) are cleavable by tumor specific MMPs. In other embodiments, cleavable peptides IVGRPRHQGV (SEQ ID NO:7), or RSKYLATA (SEQ ID NO:8) are cleavable by Cathepsin. For example, a proteolytically cleavable peptide linker comprising the cleavable peptide consisting of RPLALWRS (SEQ ID NO: 3) can be cleaved by matrix metalloproteinase-7 (MMP7).

The proteolytically cleavable peptide linker may be bonded directly or indirectly to the therapeutic moiety within the polypeptide drug construct. Where the polypeptide drug construct comprises more than one polypeptide chain, the proteolytically cleavable peptide linker may be present in the same polypeptide chain as the therapeutic moiety or in a different polypeptide chain.

The part of the construct other than the therapeutic moiety can be considered as a carrier moiety. Where the proteolytically cleavable peptide linker is covalently bonded directly to the therapeutic moiety, the proteolytically cleavable peptide linker will be located within the drug construct between the therapeutic moiety and the carrier moiety. Alternatively, the proteolytically cleavable peptide linker may be located within the carrier moiety such that the molecule that separates away after cleavage comprises the therapeutic moiety and a part of the carrier moiety.

In some embodiments, the polypeptide drug construct comprises a masking moiety. In some examples, the proteolytically cleavable peptide linker is covalently bonded directly to the therapeutic moiety and the proteolytically cleavable peptide linker is located within the drug construct between the therapeutic moiety and the masking moiety.

In some embodiments, the polypeptide drug construct comprises a targeting moiety. In some examples, the proteolytically cleavable peptide linker is covalently bonded directly to the therapeutic moiety, and the proteolytically cleavable peptide linker is located within the drug construct between the therapeutic moiety and the targeting moiety.

The polypeptide drug construct comprising one or more proteolytically cleavable peptide linkers may be a prodrug. Where the tumor-specific cleavable linker is used in a prodrug for delivering a therapeutic moiety to a tumor cell environment, the remainder of the molecule from which the therapeutic moiety separates away after cleavage may comprise a masking moiety, which inhibits the biological activity of the therapeutic moiety in the prodrug such that the therapeutic moiety is biologically active only after cleavage of the proteolytically cleavable peptide linker in the tumor cell environment. The drug construct thus may be considered to be a masked drug construct.

The masking moiety may be present in the same polypeptide chain as the therapeutic moiety. Alternatively, the masking moiety may be present in a first polypeptide chain and the therapeutic moiety may be present in a second polypeptide chain. The proteolytically cleavable peptide linker may be present in the first or second polypeptide chain.

By using a masking moiety, the systemic side effects of an administered protein therapeutic can be reduced by interfering with the binding capability of the therapeutic. By masking the therapeutic using a proteolytically cleavable peptide linker, the binding capability that is interfered with by using the masking moiety can be restored by cleavage of the proteolytically cleavable peptide linker at the tumor microenvironment. Thus, the prodrugs provided herein are engineered to precisely target pharmacological activity to the tumor microenvironment by exploiting one of the hallmarks of cancer, high local concentrations of active protease. This feature of the tumor microenvironment is used to transform a systemically inert molecule into a locally active molecules in the form of a cleavage product. Activation of the therapeutic moiety at the tumor microenvironment significantly reduces systemic toxicities that can be associated with drugs that are administered to a subject in active form.

In some embodiments, the drug construct provided herein comprises half-life extension moiety. A long half-life in vivo is important for therapeutic proteins. Unfortunately, therapeutics that are administered to a subject can have a short half-life since they are normally cleared rapidly from the subject by mechanisms including clearance by the kidney and endocytic degradation. Thus, in the drug constructs provided herein, a half-life extension moiety may be included for the purpose of extending the half-life of the therapeutic moiety in vivo. The term "half-life extension moiety" encompasses, for example, PEG, albumin, antibodies and antibody fragments. The half-life extension moiety may comprise an antibody or fragment thereof. The half-life extension moiety may comprise an Fc domain or fragment thereof. Where a polypeptide construct comprises a first half-life extension moiety and a second half-life extension moiety, and the first half-life extension moiety is associated with the second half-life extension moiety, both half-life extension moieties may comprise an Fc domain or fragment thereof.

In some embodiments, the polypeptide drug construct provided herein comprises an antibody moiety (e.g., an antibody or an antigen binding fragment thereof). In some embodiments, the polypeptide construct comprises a therapeutic moiety and a masking moiety. In some embodiments, the proteolytically cleavable peptide linker of the present invention is located between the antibody moiety and the masking moiety. In some embodiments, the proteolytically cleavable peptide linker of the present invention is located between the antibody moiety and the cytokine moiety. The antibody specifically binds to a protein, e.g., a tumor specific antigen, thereby carrying the linked therapeutic moiety to the tumor.

### 3.1 Therapeutic moieties

The polypeptide drug constructs or prodrugs described herein may comprise any therapeutic moiety.

Provided herein, in some embodiments, is a cytokine prodrug where the therapeutic moiety is a cytokine moiety. The masking moiety in the cytokine prodrug may comprise a domain of the extracellular domain of the cytokine receptor. The cytokine prodrug thus may be considered to be a masked cytokine.

The cytokine moiety may comprise a wild-type cytokine moiety or variant cytokine moiety.

The cytokine moiety may include but is not limited to IL-1, IL-2, IL-3, IL-4, IL-5, IL-6, IL-7, IL-9, IL-10, IL-11, IL-12, IL-13, IL-14, IL-15, IL-16, IL-17, IL-18, IL-20, IL-22, IL-34, TNF-α, TNF-β, CXCL8 (IL-8), G-CSF, GM-CSF, LIF, OSM, IFN-α, IFN-β, IFN-γ, CD154, LT-β, 4-1BBL, APRIL, CD70, CD153, CD178, GITRL, LIGHT, OX40L, TALL-1, TRAIL, TWEAK, TRANCE, TGF-β, M-CSF, or MSP, or a variant thereof, or a fragment thereof.

Masked cytokines (such as IL-2, IL-12 and IL-15 cytokines) are described in PCT/US2019/053588, PCT/US2021/025103, PCT/US2021/025107 and PCT/US2021/025100, which are all incorporated by reference herein.

In some examples, cytokine is IL-15.

In some examples, cytokine is IL-2.

In some examples, cytokine is IL-12.

### 3.2 Cytokine prodrugs

Cytokines play a role in cellular signalling, particularly in cells of the immune system. Provided herein is a cytokine moiety comprising a cytokine (e.g., IL-15 cytokine) or functional fragment thereof for use in a masked cytokine comprising the proteolytically cleavable peptide linker described anywhere herein, or cleavage product thereof.

### 3.2.1 'Heterodimeric' masked cytokines

Provided herein, in some embodiments, is a masked cytokine comprising a masking moiety in a first polypeptide chain and a cytokine moiety thereof in a second polypeptide chain. Such masked cytokines may be referred to as 'heterodimeric' masked cytokines.

In some embodiments, the masked cytokine comprises a protein heterodimer comprising:
a) a first polypeptide chain comprising a masking moiety linked to a first half-life extension moiety via a first linker; and
b) a second polypeptide chain comprising a cytokine moiety thereof linked to a second half-life extension moiety via a second linker,

wherein the first half-life extension moiety is associated with the second half-life extension moiety, and
wherein at least the first linker or the second linker is a proteolytically cleavable peptide linker comprising a proteolytically cleavable peptide (CP) consisting of the amino acid sequence PVSLRSGS (SEQ ID NO: 1) or GMPKDLYHAS (SEQ ID NO:2), or RPLALWRS (SEQ ID NO:3), or TQKPLGLS (SEQ ID NO:4), or APAGLIVPYN (SEQ ID NO:5), or PANLVAPDP (SEQ ID NO:6), or IVGRPRHQGV(SEQ ID NO:7), or RSKYLATA (SEQ ID NO:8).

In some embodiments, the first linker is a proteolytically cleavable peptide linker comprising a proteolytically cleavable peptide (CP) consisting of the amino acid sequence PVSLRSGS (SEQ ID NO: 1) or GMPKDLYHAS (SEQ ID NO:2), or RPLALWRS (SEQ ID NO:3), or TQKPLGLS (SEQ ID NO:4), or APAGLIVPYN (SEQ ID NO:5), or PANLVAPDP (SEQ ID NO:6), or IVGRPRHQGV(SEQ ID NO:7), or RSKYLATA (SEQ ID NO:8). The proteolytically cleavable peptide linker may be as described anywhere herein. In some embodiments, the first linker is a proteolytically cleavable peptide linker comprising a proteolytically cleavable peptide (CP) consisting of the amino acid sequence PVSLRSGS (SEQ ID NO:1). In some embodiments, the first linker is a proteolytically cleavable peptide linker comprising a proteolytically cleavable peptide (CP) consisting of the amino acid sequence GMPKDLYHAS (SEQ ID NO:2). In some embodiments, the first linker is a proteolytically cleavable peptide linker comprising a proteolytically cleavable peptide (CP) consisting of the amino acid sequence RPLALWRS (SEQ ID NO:3). In some embodiments, the first linker is a proteolytically cleavable peptide linker comprising a proteolytically cleavable peptide (CP) consisting of the amino acid sequence TQKPLGLS (SEQ ID NO:4). In some embodiments, the first linker is a proteolytically cleavable peptide linker comprising a proteolytically cleavable peptide (CP) consisting of the amino acid sequence APAGLIVPYN (SEQ ID NO:5). In some embodiments, the first linker is a proteolytically cleavable peptide linker comprising a proteolytically cleavable peptide (CP) consisting of the amino acid sequence PANLVAPDP (SEQ ID NO:6). In some embodiments, the first linker is a proteolytically cleavable peptide linker comprising a proteolytically cleavable peptide (CP) consisting of the amino acid sequence IVGRPRHQGV (SEQ ID NO:7). In some embodiments, the first linker is a proteolytically cleavable peptide linker comprising a proteolytically cleavable peptide (CP) consisting of the amino acid sequence RSKYLATA (SEQ ID NO:8). In some embodiments, the first linker is a proteolytically cleavable peptide linker and the second linker is a non-cleavable linker.

In some embodiments, the second linker is a proteolytically cleavable peptide linker comprising a proteolytically cleavable peptide (CP) consisting of the amino acid sequence PVSLRSGS (SEQ ID NO: 1) or GMPKDLYHAS (SEQ ID NO:2), or RPLALWRS (SEQ ID NO:3), or TQKPLGLS (SEQ ID NO:4), or APAGLIVPYN (SEQ ID NO:5), or PANLVAPDP (SEQ ID NO:6), or IVGRPRHQGV(SEQ ID NO:7), or RSKYLATA (SEQ ID NO:8). The proteolytically cleavable peptide linker may be as described anywhere herein. In some embodiments, the second linker is a proteolytically cleavable peptide linker comprising a proteolytically cleavable peptide (CP) consisting of the amino acid sequence PVSLRSGS (SEQ ID NO: 1). In some embodiments, the second linker is a proteolytically cleavable peptide linker comprising a proteolytically cleavable peptide (CP) consisting of the amino acid sequence GMPKDLYHAS (SEQ ID NO:2). In some embodiments, the second linker is a proteolytically cleavable peptide linker comprising a proteolytically cleavable peptide (CP) consisting of the amino acid sequence RPLALWRS (SEQ ID NO:3). In some embodiments, the second linker is a proteolytically cleavable peptide linker comprising a proteolytically cleavable peptide (CP) consisting of the amino acid sequence TQKPLGLS (SEQ ID NO:4). In some embodiments, the second linker is a proteolytically cleavable peptide linker comprising a proteolytically cleavable peptide (CP) consisting of the amino acid sequence APAGLIVPYN (SEQ ID NO:5). In some embodiments, the second linker is a proteolytically cleavable peptide linker comprising a proteolytically cleavable peptide (CP) consisting of the amino acid sequence PANLVAPDP (SEQ ID NO:6). In some embodiments, the second linker is a proteolytically cleavable peptide linker comprising a proteolytically cleavable peptide (CP) consisting of the amino acid sequence IVGRPRHQGV (SEQ ID NO:7). In some embodiments, the second linker is a proteolytically cleavable peptide linker comprising a proteolytically cleavable peptide (CP) consisting of the amino acid sequence RSKYLATA (SEQ ID NO:8). In some embodiments, the second linker is a proteolytically cleavable peptide linker and the first linker is non-cleavable. The non-cleavable linker may be as described anywhere herein.

The proteolytically cleavable peptide linker may be as described anywhere herein.

In some embodiments, in the first polypeptide chain, the first half life extension domain is linked to the amino terminus of the first linker and the carboxy terminus of the first linker is linked to the amino terminus of the masking moiety and, in the second polypeptide chain, the second half life extension domain is linked to the amino terminus of the second linker and the carboxy terminus of the second linker is linked to the amino terminus of the cytokine moiety thereof.

In some embodiments, the first polypeptide chain comprises:
**N' HL1-L1-MM** C'
and the second polypeptide chain comprises:
   **N' HL2-L2-C** C'
where HL1 is the first half life extension domain, L1 is the first linker, MM is the masking moiety, HL2 is the second half life extension domain, L2 is the second linker, and C is the cytokine moiety thereof .

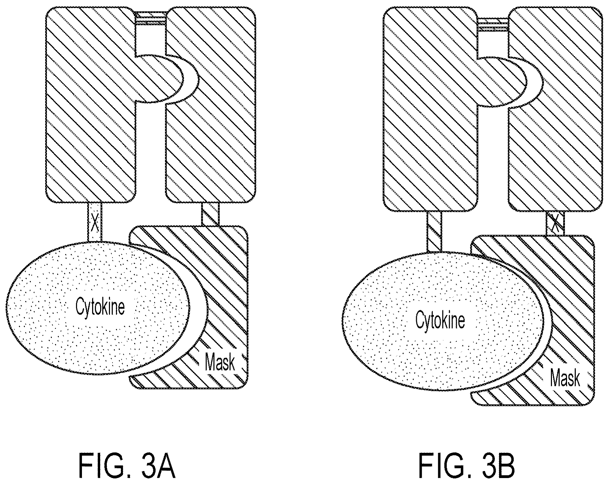

In some embodiments, the second linker is the proteolytically cleavable linker and the first linker is a non-cleavable linker. This arrangement is described herein as 'Structure A' (FIG. 3A). In some embodiments, the first polypeptide chain comprises:
**N' HL1-non-cleavable L1-MM** C'
and the second polypeptide chain comprises:
**N' HL2-cleavable L2-C** C'

In some embodiments, the first linker is the proteolytically cleavable linker and the second is a non-cleavable linker. This arrangement is described herein as 'Structure B' (FIG. 3B). In some embodiments, the first polypeptide chain comprises:
N' **HL1- cleavable L1-MM** C'
and the second polypeptide chain comprises:
N' **HL2- non-cleavable L2-C** C'

### 3.2.2 'Linear' masked cytokines

Provided herein, in some embodiments, is a masked cytokine comprising a masking moiety and a cytokine moiety thereof linked in a single polypeptide chain.

In some embodiments, the masked cytokine comprises a polypeptide chain comprising formula:

N' **HL-L2-C-L1-MM** C'

where HL is the half-life extension domain, L1 is the first linker, MM is the masking moiety, L2 is the second linker, and C is the cytokine moiety thereof, wherein at least the first linker comprises a proteolytically cleavable peptide as described anywhere herein.

In some embodiments, the masked cytokine comprises a polypeptide chain comprising formula:

N' **HL-L2-MM-L1-C** C'

where HL is the half-life extension domain, L1 is the first linker, MM is the masking moiety, L2 is the second linker, and C is the cytokine moiety thereof, wherein at least the first linker comprises a proteolytically cleavable peptide as described anywhere herein.

In some embodiments, the first linker is a cleavable linker as described anywhere herein. In some embodiments, the second linker is a non-cleavable linker.

### 3.2.3 'Targeted' cytokine

Provided herein, in some embodiments, is a masked cytokine comprising a targeting moiety, a masking moiety and a cytokine moiety and a half-life extension moiety (e.g., Fc domain). The Fc domain comprising a first Fc polypeptide linked to the cytokine moiety through a first linker and a second Fc polypeptide linked to the masking moiety through a second linker. The targeting moiety (TM) (e.g., an antibody or antigen binding domain thereof) is linked to the Fc domain through one or both of the first and second Fc polypeptides. In some embodiments, the targeting moiety comprises half-life extension properties. The first or the second linker is a cleavable linker such that the masking moiety releases the cytokine or a fragment thereof upon cleavage.

In some embodiments, the second linker between the second Fc polypeptide and the masking moiety is a cleavable linker and the first linker is a non-cleavable linker. In some embodiments, the second linker is a proteolytically cleavable peptide linker comprising a proteolytically cleavable peptide (CP) consisting of the amino acid sequence PVSLRSGS (SEQ ID NO: 1), or GMPKDLYHAS (SEQ ID NO:2), or RPLALWRS (SEQ ID NO:3), or TQKPLGLS (SEQ ID NO:4), or APAGLIVPYN (SEQ ID NO:5), or PANLVAPDP (SEQ ID NO:6), or IVGRPRHQGV(SEQ ID NO:7), or RSKYLATA (SEQ ID NO:8). The proteolytically cleavable peptide linker may be as described anywhere herein. In some embodiments, the second linker is a proteolytically cleavable peptide linker comprising a proteolytically cleavable peptide (CP) consisting of the amino acid sequence PVSLRSGS (SEQ ID NO: 1). In some embodiments, the second linker is a proteolytically cleavable peptide linker comprising a proteolytically cleavable peptide (CP) consisting of the amino acid sequence GMPKDLYHAS (SEQ ID NO:2). In some embodiments, the second linker is a proteolytically cleavable peptide linker comprising a proteolytically cleavable peptide (CP) consisting of the amino acid sequence RPLALWRS (SEQ ID NO:3). In some embodiments, the second linker is a proteolytically cleavable peptide linker comprising a proteolytically cleavable peptide (CP) consisting of the amino acid sequence TQKPLGLS (SEQ ID NO:4). In some embodiments, the second linker is a proteolytically cleavable peptide linker comprising a proteolytically cleavable peptide (CP) consisting of the amino acid sequence APAGLIVPYN (SEQ ID NO:5). In some embodiments, the second linker is a proteolytically cleavable peptide linker comprising a proteolytically cleavable peptide (CP) consisting of the amino acid sequence PANLVAPDP (SEQ ID NO:6). In some embodiments, the second linker is a proteolytically cleavable peptide linker comprising a proteolytically cleavable peptide (CP) consisting of the amino acid sequence IVGRPRHQGV (SEQ ID NO:7). In some embodiments, the second linker is a proteolytically cleavable peptide linker comprising a proteolytically cleavable peptide (CP) consisting of the amino acid sequence RSKYLATA (SEQ ID NO:8).

In some embodiments, the first linker between the first Fc polypeptide and the cytokine moiety is a cleavable linker and the second linker is a non-cleavable linker. In some embodiments, the first linker is a proteolytically cleavable peptide linker comprising a proteolytically cleavable peptide (CP) consisting of the amino acid sequence PVSLRSGS (SEQ ID NO:1), or GMPKDLYHAS (SEQ ID NO:2), or RPLALWRS (SEQ ID NO:3), or TQKPLGLS (SEQ ID NO:4), or APAGLIVPYN (SEQ ID NO:5), or PANLVAPDP (SEQ ID NO:6), or IVGRPRHQGV(SEQ ID NO:7), or RSKYLATA (SEQ ID NO:8). In some embodiments, the first linker is a proteolytically cleavable peptide linker comprising a proteolytically cleavable peptide (CP) consisting of the amino acid sequence PVSLRSGS (SEQ ID NO: 1). In some embodiments, the first linker is a proteolytically cleavable peptide linker comprising a proteolytically cleavable peptide (CP) consisting of the amino acid sequence GMPKDLYHAS (SEQ ID NO:2). In some embodiments, the first linker is a proteolytically cleavable peptide linker comprising a proteolytically cleavable peptide (CP) consisting of the amino acid sequence RPLALWRS (SEQ ID NO:3). In some embodiments, the first linker is a proteolytically cleavable peptide linker comprising a proteolytically cleavable peptide (CP) consisting of the amino acid sequence TQKPLGLS (SEQ ID NO:4). In some embodiments, the first linker is a proteolytically cleavable peptide linker comprising a proteolytically cleavable peptide (CP) consisting of the amino acid sequence APAGLIVPYN (SEQ ID NO:5). In some embodiments, the first linker is a proteolytically cleavable peptide linker comprising a proteolytically cleavable peptide (CP) consisting of the amino acid sequence PANLVAPDP (SEQ ID NO:6). In some embodiments, the first linker is a proteolytically cleavable peptide linker comprising a proteolytically cleavable peptide (CP) consisting of the amino acid sequence IVGRPRHQGV (SEQ ID NO:7). In some embodiments, the first linker is a proteolytically cleavable peptide linker comprising a proteolytically cleavable peptide (CP) consisting of the amino acid sequence RSKYLATA (SEQ ID NO:8).

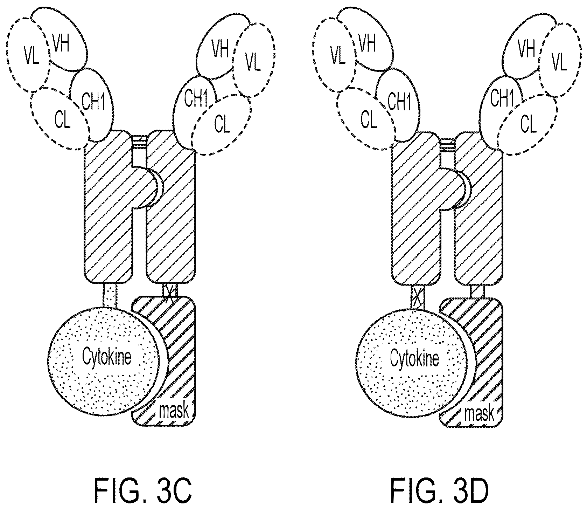

In some embodiments, the targeting moiety (TM) second linker is the proteolytically cleavable linker and the first linker is a non-cleavable linker. This arrangement is described herein as 'Structure C' (FIG. 3C). In some embodiments, the first polypeptide chain comprises:
**N' TM1-non-cleavable L1-MM C'**
and the second polypeptide chain comprises:
N' **TM2-cleavable L2-C** C'
where TM1 and TM2 are the targeting moieties, L1 is the first linker, MM is the masking moiety, L2 is the second linker, and C is the cytokine moiety.

In some embodiments, the first linker is the proteolytically cleavable linker and the second is a non-cleavable linker. This arrangement is described herein as 'Structure D' (FIG. 3D). In some embodiments, the first polypeptide chain comprises:
N' **TM1- cleavable L1-MM** C'
and the second polypeptide chain comprises:
N' **TM2- non-cleavable L2-C** C'
where TM1 and TM2 are the targeting moieties, L1 is the first linker, MM is the masking moiety, L2 is the second linker, and C is the cytokine moiety.

### 4. CLEAVAGE PRODUCT

Provided herein is a cleavage product, preparable by proteolytic cleavage of the proteolytically cleavable linker in the polypeptide constructs as described anywhere herein.

Upon proteolytic cleavage of the cleavable linker at the cleavage site, a cleavage product is formed.

Also provided herein is a cleavage product capable of comprising an active therapeutic moiety, preparable by proteolytic cleavage of the proteolytically cleavable linker in polypeptide drug construct as described anywhere herein. Where said polypeptide drug construct is a masked cytokine as described anywhere herein, upon proteolytic cleavage, a cleavage product comprising the cytokine moiety is formed. The cytokine moiety in the cleavage product is activated since it is no longer masked by the masking moiety. The cytokine moiety in the cleavage product is therefore capable of binding to the target protein.

Provided herein is a cleavage product of a 'heterodimeric' masked cytokine described anywhere herein.

Provided herein is a cleavage product of a 'linear' masked cytokine described anywhere herein.

Provided herein is a cleavage product of a "targeted' cytokine described anywhere herein.

The tumor cell environment is complex and can comprise multiple different proteases. As such, the precise site at which a given cleavable peptide within a polypeptide construct will be cleaved in the tumor cell environment may vary between tumor types, between patients with the same tumor type and even between cleavage products formed in the same tumor. Moreover, even after cleavage, further modification of the initial cleavage product, e.g. by removal of one or two terminal amino acids, may occur by the further action of proteases in the tumor cell environment. A distribution of cleavage products can thus be expected to form in the tumor cell environment of a patient following administration of a polypeptide construct as described herein.

It will be understood that a cleavage site as referred to herein refers to a site between two specific amino acid residues within the cleavable peptide that are a target for a protease known to be associated with a tumor cell environment. In this sense, there may be more than one cleavage site present in a cleavable peptide as described herein where different proteases cleave the cleavable peptide at different cleavage sites. It is also possible that more than one protease may act on the same cleavage site within a cleavable peptide. Discussion of protease cleavage sites can be found in the art.

Thus, the cleavable peptides disclosed herein may be cleaved by one or more proteases.

Provided herein is a cleavage product comprising a cytokine moiety capable of binding to it cognate receptor, preparable by proteolytic cleavage of the proteolytically cleavable linker in a masked cytokine as described anywhere herein.

Also provided herein is a distribution of cleavage products obtained or obtainable from a single structure of a masked cytokine, where each cleavage product within the distribution of cleavage products (i) is capable of binding to the target protein and (ii) comprises a cytokine (e.g., IL-15 cytokine) moiety as defined anywhere herein.

The location of the cleavable peptide in the polypeptide construct determines the structure of the resulting cleavage product.

It will be understood that the cleavage product may comprise a portion of the proteolytically cleavable peptide.

A "portion of a proteolytically cleavable peptide", refers to a part of the original proteolytically cleavable peptide sequence after cleavage at the cleavage site has occurred. After cleavage, further modification of the initial cleavage product, e.g. by removal of one or two terminal amino acids, may also occur by the further action of e.g. proteases in the tumor cell environment. As such, cleavage products within the distribution of cleavage products that might be formed might not contain any portion of the proteolytically cleavable peptide.

In some embodiments, a "portion" refers to 1 amino acid, 2 amino acids, 3 amino acids, 4 amino acids, 5 amino acids or 6 amino acids of the original proteolytically cleavable peptide sequence. In some embodiments, a "portion" refers to 2 amino acids of the original proteolytically cleavable peptide sequence. In some embodiments, a "portion" refers to 3 amino acids of the original proteolytically cleavable peptide sequence. In some embodiments, a "portion" refers to 4 amino acids of the original proteolytically cleavable peptide sequence.

In some embodiments, the 'portion' of the proteolytically cleavable peptide is from 3 to 6 amino acids in length. In some embodiments, the 'portion' of the proteolytically cleavable peptide is 3 or 4 amino acids in length.

Exemplary cleavage sites for cleavable linkers disclosed herein are disclosed herein **PVSL*RSGS** (SEQ ID NO:1) and **GMPKDLYHA*S** (SEQ ID NO:2) (* indicates a known or observed protease cleavage site within the cleavable peptide).

Accordingly, herein disclosed is the cleavage product of any one of the polypeptide constructs disclosed herein.

### 5. COMPOSITIONS

In some aspects, also provided herein are compositions comprising any of the polypeptide constructs described herein. In some embodiments, the composition comprises any of the drug constructs described herein. In some embodiments, the composition comprises any of the masked drug constructs described herein. In some embodiments, the composition comprises any of the masked cytokines described herein.

In some embodiments, the composition is a pharmaceutical composition.

In some embodiments, the composition comprises a polypeptide construct as described herein and further comprises one or more components. For example, in some embodiments, the composition comprises one or more pharmaceutically acceptable carriers, excipients, stabilizers, buffers, preservatives, tonicity agents, non-ionic surfactants or detergents, or other therapeutic agents or active compounds, or combinations thereof. The various embodiments of the composition are sometimes referred to herein as formulations.

### 6. METHODS OF TREATMENT

Provided herein are methods for treating or preventing a disease in a subject comprising administering to the subject an effective amount of any drug construct described herein, for example any masked cytokine, or compositions thereof. In some embodiments, methods are provided for treating or preventing a disease in a subject comprising administering to the subject any composition described herein. In some embodiments, the subject (e.g., a human patient) has been diagnosed with cancer or is at risk of developing such a disorder. In some embodiments, methods are provided for treating or preventing disease in a subject comprising administering to the subject an effective amount of any masked drug construct described herein or compositions thereof, wherein the masked drug construct is activated upon cleavage by an enzyme. In some embodiments, the masked drug construct is activated at a tumor microenvironment. The masked drug construct is therapeutically active after it has cleaved. Thus, in some embodiments, the active agent is the cleavage product.

For the prevention or treatment of disease, the appropriate dosage of an active agent will depend on the type of disease to be treated, as defined herein, the severity and course of the disease, whether the agent is administered for preventive or therapeutic purposes, previous therapy, the subject's clinical history and response to the agent, and the discretion of the attending physician. The agent is suitably administered to the subject at one time or over a series of treatments.

In some embodiments, the protease acting to cleave the proteolytically cleavable peptide is a MMP. In some embodiments, the MMP is selected from the group consisting of MMP-1, MMP-2, MMP-3, MMP-7, MMP-8, MMP-9, MMP-10, MMP-11, MMP-12, MMP-13, MMP-14, MMP-15, MMP-16, MMP-17, MMP-19, MMP-20, MMP-21, MMP-23A, MMP-23B, MMP-24, MMP-25, MMP-27, MMP-28. In some embodiments, the protease acting to cleave the proteolytically cleavable peptide is MMP-2. In some embodiments, the protease acting to cleave the proteolytically cleavable peptide is MMP-7. In some embodiments, the protease acting to cleave the proteolytically cleavable peptide is MMP-8. In some embodiments, the protease acting to cleave the proteolytically cleavable peptide is MMP-9. In some embodiments, the protease acting to cleave the proteolytically cleavable peptide is MMP-10. In some embodiments, the protease acting to cleave the proteolytically cleavable peptide is MMP-14.

In some embodiments, the protease acting to cleave the proteolytically cleavable peptide is cathepsin, e.g., Cathepsin B, cathepsin D, cathepsin F, cathepsin K, cathepsin L, cathepsin V, cathepsin S, and cathepsin W. In some embodiments, the protease acting to cleave the proteolytically cleavable peptide is Cathepsin B (CatB).

In some embodiments, provided herein is a method of treatment or prevention of a cancer by administration of any masked drug construct, for example masked cytokine, or composition described herein in combination with an anticancer therapeutic protein. The anti-cancer therapeutic protein can be any therapeutic protein capable of reducing cancer growth, interfering with cancer cell replication, directly or indirectly killing cancer cells, reducing metastasis, reducing tumor blood supply, or reducing cell survival. Exemplary anti-cancer therapeutic proteins may come in the form of an antibody or fragment thereof, an antibody derivative, a bispecific antibody, a chimeric antigen receptor (CAR) T cell, a fusion protein, or a bispecific T-cell engager (BiTE). In some embodiments, provided herein is a method of treatment or prevention of a cancer by administration of any masked cytokine or composition described herein in combination with CAR-NK (Natural Killer) cells.

### 7. ARTICLES OF MANUFACTURE OR KITS

In another aspect, an article of manufacture or kit is provided which comprises any polypeptide construct described herein. The article of manufacture or kit may further comprise instructions for use of the polypeptides in the methods of the invention, for example instructions for use of masked cytokines in the method of the invention. Thus, in certain embodiments, the article of manufacture or kit comprises instructions for the use of a masked cytokine in methods for treating or preventing a disorder (e.g., a cancer) in an individual comprising administering to the individual an effective amount of a masked cytokine. For example, in certain embodiments, the article of manufacture or kit comprises instructions for the use of a masked polypeptide in methods for treating or preventing a disorder (e.g., a cancer) in an individual comprising administering to the individual an effective amount of a masked polypeptide. In certain embodiments, the individual is a human. In some embodiments, the individual has a disease selected from the group consisting of include leukemia, lymphoma, head and neck cancer, colorectal cancer, prostate cancer, pancreatic cancer, melanoma, breast cancer, neuroblastoma, lung cancer, ovarian cancer, osteosarcoma, bladder cancer, cervical cancer, liver cancer, kidney cancer, skin cancer or testicular cancer.

The article of manufacture or kit may further comprise a container. The container holds the formulation. In some embodiments, the formulation is a lyophilized formulation. In some embodiments, the formulation is a frozen formulation. In some embodiments, the formulation is a liquid formulation.

The article of manufacture or kit may further comprise a label or a package insert, which is on or associated with the container, may indicate directions for reconstitution and/or use of the formulation.

The article of manufacture or kit herein optionally further comprises a container comprising a second medicament, wherein the masked drug construct is a first medicament, and which article or kit further comprises instructions on the label or package insert for treating the subject with the second medicament, in an effective amount.

In another embodiment, provided herein is an article of manufacture or kit comprising the formulations described herein for administration in an auto-injector device.

### 8. DEFINITIONS

Unless defined otherwise, all terms of art, notations and other technical and scientific terms or terminology used herein are intended to have the same meaning as is commonly understood by one of ordinary skill in the art to which the claimed subject matter pertains. In some cases, terms with commonly understood meanings are defined herein for clarity and/or for ready reference, and the inclusion of such definitions herein should not necessarily be construed to represent a substantial difference over what is generally understood in the art.

It is to be understood that this invention is not limited to particular compositions or biological systems, which can, of course, vary. It is also to be understood that the terminology used herein is for the purpose of describing particular embodiments only, and is not intended to be limiting. As used in this specification and the appended claims, the singular forms "a," "an," and "the" include plural referents unless the content clearly dictates otherwise.

The term "about" as used herein refers to the usual error range for the respective value readily known to the skilled person in this technical field. Reference to "about" a value or parameter herein includes (and describes) embodiments that are directed to that value or parameter per se.

It is understood that aspects and embodiments of the invention described herein include "comprising," "consisting," and "consisting essentially of' aspects and embodiments.

As used herein, the term "and/or" refers to any one of the items, any combination of the items, or all of the items with which the term is associated. For instance, the phrase "A, B, and/or C" is intended to encompass each of the following embodiments: A, B, and C; A, B, or C; A or B; A or C; B or C; A and B; A and C; B and C; A and B or C; B and A or C; C and A or B; A (alone); B (alone); and C (alone).

The term "antibody" includes polyclonal antibodies, monoclonal antibodies (including full length antibodies which have an immunoglobulin Fc region), antibody compositions with polyepitopic specificity, multispecific antibodies (e.g., bispecific antibodies, diabodies, and single-chain molecules, as well as antibody fragments (e.g., Fab, F(ab')2, and Fv). The term "immunoglobulin" (Ig) is used interchangeably with "antibody" herein.

The terms "full-length antibody," "intact antibody" or "whole antibody" are used interchangeably to refer to an antibody in its substantially intact form, as opposed to an antibody fragment. Specifically, whole antibodies include those with heavy and light chains including an Fc region. The constant domains may be native sequence constant domains (e.g., human native sequence constant domains) or amino acid sequence variants thereof. In some cases, the intact antibody may have one or more effector functions.

An "antibody fragment" comprises a portion of an intact antibody, such as the antigen binding region and/or the variable region of the intact antibody, and/or the constant region of the intact antibody. Examples of an antibody fragment include the Fc region of the antibody, a portion of the Fc region, or a portion of the antibody comprising the Fc region. Examples of antigen-binding antibody fragments include domain antibodies (dAbs), Fab, Fab', F(ab')2 and Fv fragments; diabodies; linear antibodies (see U.S. Pat. No. 5,641,870, Example 2; Zapata et ah, Protein Eng. 8(10): 1057-1062[1995]); single-chain antibody molecules, and multispecific antibodies formed from antibody fragments. Single heavy chain antibodies or single light chain antibodies can be engineered, or in the case of the heavy chain, can be isolated from camelids, shark, libraries or mice engineered to produce single heavy chain molecules.

The term "pharmaceutical formulation" refers to a preparation that is in such form as to permit the biological activity of the active ingredient to be effective, and that contains no additional components that are unacceptably toxic to a subject to which the formulation would be administered. Such formulations are sterile.

"Carriers" as used herein include pharmaceutically acceptable carriers, excipients, or stabilizers that are nontoxic to the cell or mammal being exposed thereto at the dosages and concentrations employed. Often the physiologically acceptable carrier is an aqueous pH buffered solution. Examples of physiologically acceptable carriers include buffers such as phosphate, citrate, and other organic acids; antioxidants including ascorbic acid; low molecular weight (less than about 10 residues) polypeptide; proteins, such as serum albumin, gelatin, or immunoglobulins; hydrophilic polymers such as polyvinylpyrrolidone; amino acids such as glycine, glutamine, asparagine, arginine or lysine; monosaccharides, disaccharides, and other carbohydrates including glucose, mannose, or dextrins; chelating agents such as EDTA; sugar alcohols such as mannitol or sorbitol; salt-forming counterions such as sodium; and/or nonionic surfactants such as TWEEN^{™}, polyethylene glycol (PEG), and PLURONICS^{™}.

As used herein, the term "treatment" refers to clinical intervention designed to alter the natural course of the individual or cell being treated during the course of clinical pathology. Desirable effects of treatment include decreasing the rate of disease progression, ameliorating or palliating the disease state, and remission or improved prognosis. An individual is successfully "treated", for example, if one or more symptoms associated with a disorder (e.g., a neoplastic disease) are mitigated or eliminated. For example, an individual is successfully "treated" if treatment results in increasing the quality of life of those suffering from a disease, decreasing the dose of other medications required for treating the disease, reducing the frequency of recurrence of the disease, lessening severity of the disease, delaying the development or progression of the disease, and/or prolonging survival of individuals.

As used herein, "in conjunction with" or "in combination with" refers to administration of one treatment modality in addition to another treatment modality. As such, "in conjunction with" or "in combination with" refers to administration of one treatment modality before, during or after administration of the other treatment modality to the individual.

As used herein, the term "prevention" includes providing prophylaxis with respect to occurrence or recurrence of a disease in an individual. An individual may be predisposed to, susceptible to a disorder, or at risk of developing a disorder, but has not yet been diagnosed with the disorder. In some embodiments, masked cytokines described herein are used to delay development of a disorder.

As used herein, an individual "at risk" of developing a disorder may or may not have detectable disease or symptoms of disease, and may or may not have displayed detectable disease or symptoms of disease prior to the treatment methods described herein. "At risk" denotes that an individual has one or more risk factors, which are measurable parameters that correlate with development of the disease, as known in the art. An individual having one or more of these risk factors has a higher probability of developing the disorder than an individual without one or more of these risk factors.

An "effective amount" refers to at least an amount effective, at dosages and for periods of time necessary, to achieve the desired or indicated effect, including a therapeutic or prophylactic result.

An effective amount can be provided in one or more administrations. A "therapeutically effective amount" is at least the minimum concentration required to effect a measurable improvement of a particular disorder. A therapeutically effective amount herein may vary according to factors such as the disease state, age, sex, and weight of the patient, and the ability of the antibody to elicit a desired response in the individual. A therapeutically effective amount may also be one in which any toxic or detrimental effects of the masked cytokine are outweighed by the therapeutically beneficial effects. A "prophylactically effective amount" refers to an amount effective, at the dosages and for periods of time necessary, to achieve the desired prophylactic result. Typically, but not necessarily, since a prophylactic dose is used in subjects prior to or at the earlier stage of disease, the prophylactically effective amount can be less than the therapeutically effective amount.

"Chronic" administration refers to administration of the medicament(s) in a continuous as opposed to acute mode, so as to main the initial therapeutic effect (activity) for an extended period of time. "Intermittent" administration is treatment that is not consecutively done without interruption, but rather is cyclic in nature.

As used herein, an "individual" or a "subject" is a mammal. A "mammal" for purposes of treatment includes humans, domestic and farm animals, and zoo, sports, or pet animals, such as dogs, horses, rabbits, cattle, pigs, hamsters, gerbils, mice, ferrets, rats, cats, etc. In some embodiments, the individual or subject is human.

### EXAMPLES

The invention will be more fully understood by reference to the following examples. They should not, however, be construed as limiting the scope of the invention. It is understood that the examples and embodiments described herein are for illustrative purposes only and that various modifications or changes in light thereof will be suggested to persons skilled in the art and are to be included within the spirit and purview of this application and scope of the appended claims.

Although some examples describe the engineering, production, and/or testing of "masked" versions of an polypeptide construct, some examples also employ parental "non-masked" versions of the polypeptide construct, such as for comparison, or other constructs that include one or more of the components described herein that are tested as controls for comparison. Accordingly, the description of, for instance, testing done on masked polypeptide constructs does not necessarily mean that non-masked versions of the construct were not also tested.

### Example 1: Cleavage of peptides by NAT vs. RCC culture supernatant

Sequences comprising cleavage peptides PVSLRSGS (SEQ ID NO:1) or GMPKDLYHAS (SEQ ID NO:2) (shown in bold below) were incubated in either 'NAT' (Normal Adjacent Tissue) or 'RCC' (Renal Cell Carcinoma) culture supernatants, to test the specificity of each peptide's cleavage. Carfilzomib (CFZ) is also included in the incubation media as an inhibitor of proteosome, to inhibit false positive cleavage.

To this end, peptide sequencing by mass spectrometry was used to identify cleaved fragments produced for the synthetic peptides shown in the table below, using a published technique called multiplexed substrate profiling by mass spectrometry (MSP-MS) (O'Donoghue A.J. et al. Nat Methods. 2012 Nov;9(11):1095-100.) Cleavages were monitored in these reactions over time, and the peptides found to be cleaved in the earliest time points were deemed to be most sensitive to proteolytic activity in the conditioned media samples. Results are as follows:

| Substrate | Synthetic Tissue Sequence (bolded sequences show the cleavable peptide; * indicates cleavage site) | NAT + CFZ | RCC + CFZ | Earliest cleaved time point - NAT | Earliest cleaved time point - RCC |
|---|---|---|---|---|---|
| Alu-XT-019 | **RVGPVSL*R*SGS**GKI (SEQ ID NO: 9) | 1/5 | 5/5 | 5min | 15min |
| Alu-XT-091 | **RQGGM*PKDLYHA*SAKQ** (SEQ ID NO: 10) | 1/5 | 5/5 | 5min | 15min |

### Example 2: Heterodimeric masked cytokines and targeted cytokines

This example demonstrates heterodimeric masked cytokines designed to incorporate a proteolytically cleavable peptide (CP) consisting of the amino acid sequence PVSLRSGS (SEQ ID NO: 1) or GMPKDLYHAS (SEQ ID NO:2) , or RPLALWRS (SEQ ID NO: 3), or TQKPLGLS (SEQ ID NO:4), or APAGLIVPYN (SEQ ID NO:5), or PANLVAPDP (SEQ ID NO:6), or IVGRPRHQGV (SEQ ID NO:7), or RSKYLATA (SEQ ID NO:8). Heterodimeric masked cytokines described in this example include a cleavable peptide on one polypeptide chain. Specifically masked cytokines with the following configurations are generated:

**Table 1: Masked cytokines with cleavable linkers**

| | | | |
|---|---|---|---|
| | First Polypeptide Chain | Second Polypeptide Chain | Cleavage Position |
| Structure A (Figure 3A) | N' HL1-non-cleavable L1-MM C' | N' HL2-cleavable L2-C C' | Cleavage Site (PVSLRSGS or GMPKDLYHAS, or RPLALWRS, or TQKPLGLS, or APAGLIVPYN, or PANLVAPDP, or IVGRPRHQGV, or RSKYLATA) on Cytokine arm |
| Structure B (Figure 3B) | N' HL1- cleavable L1-MM C' | N' HL2- non-cleavable L2-C C' | Cleavage Site (PVSLRSGS or GMPKDLYHAS, , or RPLALWRS, or TQKPLGLS, or APAGLIVPYN, or PANLVAPDP, or IVGRPRHQGV, or RSKYLATA) on masking arm |
| Structure C (Figure 3C) | N' TM1-HL1-cleavable L1-MM C' | N' TM2-HL2-non-cleavable L2-C C' | Cleavage Site (PVSLRSGS or GMPKDLYHAS, , or RPLALWRS, or TQKPLGLS, or APAGLIVPYN, or PANLVAPDP, or IVGRPRHQGV, or RSKYLATA) on masking arm |
| Structure D (Figure 3D) | N' TM1-HL1-non-cleavable L1-MM C' | N' TM2-HL2-cleavable L2-C C' | Cleavage Site (PVSLRSGS or GMPKDLYHAS, , or RPLALWRS, or TQKPLGLS, or APAGLIVPYN, or PANLVAPDP, or IVGRPRHQGV, or RSKYLATA) on cytokine arm |

HL1 is the first half life extension domain (e.g., Fc), L1 is the first linker, MM is the masking moiety (e.g., a cognate receptor for the cytokine such as CD122), HL2 is the second half life extension domain (e.g. Fc), L2 is the second linker, and C is the cytokine moiety (e.g., IL-15). TM is a targeting moiety (e.g., an antibody or an antigen binding domain thereof). Cleavage capacity of the heterodimeric constructs described above that incorporate PVSLRSGS or GMPKDLYHAS cleavage peptides are incubated with MMP7, 9 and 10 and analysed by SDS-PAGE. Cleavage is confirmed by HEK-Blue IL-2 bioassay. Briefly, constructs are diluted to 1uM with PBS. Recombinant cytokine (e.g., IL-15) at 0.1 mg/ml is used as a control. HEK-Blue cells are seeded with 50K cells/well at 150 uL per well in a 96 well plate. Constructs or recombinant cytokine are incubated at 37°C in 5% CO2 for 24 hours. 180uL QUANTI-blue (QB) solution and 20uL cell supernatant is incubated at 37°C for 1 hour. Results are read at 625nM on a plate reader to determine specific EC50.

### Example 3: Identification of new cleavable peptides

This example illustrates the discovery of 7 protease substrates, which are more cleaved by tumor than by plasma from RCC, and Head and Neck (H&N) cancer patients, and highly cleaved by recombinant MMP or Cathepsin B.

**Table 2: New protease substrates**

| **Peptide sequence** | **SEQ ID NO** |
|---|---|
| **P**VSLRSGS | SEQ ID NO: 1 |
| RPLA**L**WRS | SEQ ID NO:3 |
| TQK**P**LGLS | SEQ ID NO:4 |
| A**P**AGLIVPYN | SEQ ID NO:5 |
| **P**ANLVAPDP | SEQ ID NO:6 |
| **IVGRPRHQGV** | SEQ ID NO:7 |
| **RSKYLATA** | SEQ ID NO:8 |

In this example, the peptides library was subjected to cleavage screening by biological matrices, i.e., tumor conditioned media (TCM). The cleavage of the peptides library by plasma was performed for comparison. Each peptide in the library includes a fluorescent mark at one end of its sequence and a quencher on the other end of the sequence. Upon cleavage of the peptide, the quencher is separated from the fluorescent mark, thereby releasing the fluorescent mark. The peptide substrates with the highest ratio of (initial rate of cleavage by tumor conditioned media (TCM) - initial rate of cleavage by plasma) in pmol/min, from cancer patients were identified.

**Table 3: Ratio of Initial rate of cleavage by tumor conditioned media (TCM) - initial rate of cleavage by plasma**

| **Peptide sequence** | **RCC TCM-Plasma** | **H&N TCM --Plasma** |
|---|---|---|
| RPLALWRS (SEQ ID NO: 3) | 0.5 | 0.0 |
| TQKPLGLS (SEQ ID NO: 4) | 0.2 | 0.2 |
| APAGLIVPYN (SEQ ID NO: 5) | 0.2 | 0.1 |
| PVSLRSGS (SEQ ID NO: 1) | 0.2 | 0.0 |
| PANLVAPDP (SEQ ID NO: 6) | 0.1 | 0.4 |
| IVGRPRHQGV (SEQ ID NO: 7) | 0.6 | 0.1 |
| RSKYLATA (SEQ ID NO: 8) | 0.3 | 0.0 |

The peptides with high ratio of initial rate of cleavage by tumor conditioned media (TCM) to initial rate of cleavage by plasma were further screened using recombinant proteases. The peptide substrates that have high initial rates (uM/h) of cleavage by recombinant proteases were identified (Table 4).

**Table 4: Rates of cleavage by recombinant proteases**

| **Peptide sequence** | **MMP-1** | **MMP-2** | **MMP-7** | **MMP-8** | **MMP-9** | **MMP-10** | **MMP-14** | **CatB** |
|---|---|---|---|---|---|---|---|---|
| RPLALWRS (SEQ ID NO: 3) | 0.1 | 1.1 | 0.2 | 0.2 | 0.2 | 0.0 | 0.0 | 0.3 |
| TQKPLGLS (SEQ ID NO: 4) | 0.0 | 11.8 | 1.8 | 0.1 | 0.3 | 0.0 | 0.1 | 0.3 |
| APAGLIVPYN (SEQ ID NO: 5) | 0.0 | 29.6 | 2.4 | 0.5 | 1.8 | 1.6 | 2.1 | 1.0 |
| PVSLRSGS (SEQ ID NO: 1) | 0.0 | 1.9 | 0.2 | 0.1 | 6.5 | 0.0 | 0.0 | 0.0 |
| PANLVAPDP (SEQ ID NO: 6) | 2.7 | 31.2 | 2.5 | 7.2 | 8.8 | 1.8 | 0.7 | 0.0 |
| IVGRPRHQGV (SEQ ID NO: 7) | 0.0 | 0.0 | 0.0 | 0.0 | 0.0 | 0.0 | 0.0 | 1.9 |
| RSKYLATA (SEQ ID NO: 8) | 0.0 | 0.0 | 0.0 | 0.0 | 0.0 | 0.0 | 0.0 | 1.9 |

### Equivalents and Scope

The present invention is not intended to be limited in scope to the particular disclosed embodiments, which are provided, for example, to illustrate various aspects of the invention. Various modifications to the compositions and methods described will become apparent from the description and teachings herein. Such variations may be practiced without departing from the true scope and spirit of the disclosure and are intended to fall within the scope of the present disclosure.

### Aspects of the invention are disclosed in the following numbered clauses:

1. A polypeptide comprising a proteolytically cleavable peptide linker, wherein the linker comprises a proteolytically cleavable peptide (CP) consisting of the amino acid sequence PVSLRSGS (SEQ ID NO: 1) or GMPKDLYHAS (SEQ ID NO:2), or RPLALWRS (SEQ ID NO:3), or TQKPLGLS (SEQ ID NO:4), or APAGLIVPYN (SEQ ID NO:5), or PANLVAPDP (SEQ ID NO:6), or IVGRPRHQGV (SEQ ID NO:7), or RSKYLATA (SEQ ID NO:8).
2. A polypeptide construct comprising a proteolytically cleavable peptide linker, wherein the linker comprises a proteolytically cleavable peptide (CP) consisting of the amino acid sequence PVSLRSGS (SEQ ID NO:1) or GMPKDLYHAS (SEQ ID NO:2), or RPLALWRS (SEQ ID NO:3), or TQKPLGLS (SEQ ID NO:4), or APAGLIVPYN (SEQ ID NO:5), or PANLVAPDP (SEQ ID NO:6), or IVGRPRHQGV (SEQ ID NO:7), or RSKYLATA (SEQ ID NO:8).
3. The polypeptide or polypeptide construct according to clause 1 or 2, wherein the proteolytically cleavable peptide (CP) is PVSLRSGS (SEQ ID NO: 1).
4. The polypeptide or polypeptide construct according to clause 1 or 2, wherein the proteolytically cleavable peptide (CP) is GMPKDLYHAS (SEQ ID NO:2).
5. The polypeptide or polypeptide construct according to clause 1 or 2, wherein the proteolytically cleavable peptide (CP) is RPLALWRS (SEQ ID NO:3).
6. The polypeptide or polypeptide construct according to clause 1 or 2, wherein the proteolytically cleavable peptide (CP) is TQKPLGLS (SEQ ID NO:4).
7. The polypeptide or polypeptide construct according to clause 1 or 2, wherein the proteolytically cleavable peptide (CP) is APAGLIVPYN (SEQ ID NO:5).
8. The polypeptide or polypeptide construct according to clause 1 or 2, wherein the proteolytically cleavable peptide (CP) is PANLVAPDP (SEQ ID NO:6).
9. The polypeptide or polypeptide construct according to clause 1 or 2, wherein the proteolytically cleavable peptide (CP) is IVGRPRHQGV (SEQ ID NO:7).
10. The polypeptide or polypeptide construct according to clause 1 or 2, wherein the proteolytically cleavable peptide (CP) is RSKYLATA (SEQ ID NO:8).
11. The polypeptide or polypeptide construct according to any one of clauses 1 to 10, wherein the proteolytically cleavable peptide linker is from 8 to 25 amino acids in length.
12. The polypeptide or polypeptide construct according to any one of clauses 1 to 11, wherein the proteolytically cleavable peptide linker is from 10 to 25 amino acids in length.
13. The polypeptide construct according to any one of clauses 2 to 12, wherein the polypeptide construct comprises a therapeutic moiety.
14. The polypeptide construct according to any one of clauses 2 to 13, wherein the proteolytically cleavable peptide linker is located within the construct between a therapeutic moiety and a carrier moiety.
15. The polypeptide construct according to any one of clauses 2 to 14, wherein the polypeptide construct comprises a single polypeptide chain.
16. The polypeptide construct according to any one of clauses 2 to 15, wherein the polypeptide construct comprises more than one polypeptide chain.
17. The polypeptide construct according to clause 16, wherein the proteolytically cleavable peptide linker is present in the same polypeptide chain as the therapeutic moiety.
18. The polypeptide construct according to clause 16, wherein the proteolytically cleavable peptide linker is present in a different polypeptide chain to the therapeutic moiety.
19. The polypeptide construct according to any one of clauses 2 to 18, wherein the polypeptide construct is a prodrug.
20. The polypeptide construct according to any one of clauses 2 to 19, further comprising a masking moiety.
21. The polypeptide construct according to any one of clauses 2 to 20, further comprising a half-life extension moiety.
22. The polypeptide construct according to any one of clauses 2 to 21, further comprising a targeting moiety.
23. A cytokine prodrug according to clause 19, wherein the cytokine prodrug comprises a masking moiety and the masking moiety comprises a domain of the extracellular domain of the cytokine receptor.
24. A masked cytokine comprising:
   a) a first polypeptide chain comprising a masking moiety linked to a first half-life extension moiety via a first linker; and
   b) a second polypeptide chain comprising a cytokine moiety thereof linked to a second half-life extension moiety via a second linker,

   wherein the first half-life extension moiety is associated with the second half-life extension moiety, and
   wherein at least the first linker or the second linker is a proteolytically cleavable peptide linker comprising a proteolytically cleavable peptide (CP) consisting of the amino acid sequence PVSLRSGS (SEQ ID NO: 1) or GMPKDLYHAS (SEQ ID NO:2), or RPLALWRS (SEQ ID NO:3), or TQKPLGLS (SEQ ID NO:4), or APAGLIVPYN (SEQ ID NO:5), or PANLVAPDP (SEQ ID NO:6), or IVGRPRHQGV (SEQ ID NO:7), or RSKYLATA (SEQ ID NO:8).
25. The masked cytokine according to clause 24, wherein the second linker is the proteolytically cleavable linker and the first linker is a non-cleavable linker.
26. The masked cytokine according to clause 24, wherein the first linker is the proteolytically cleavable linker and the second linker is a non-cleavable linker.
27. The masked cytokine according to any one of clauses 24 to 26, wherein proteolytically cleavable peptide (CP) consists of the amino acid sequence PVSLRSGS (SEQ ID NO:1).
28. The masked cytokine according to any one of clauses 24 to 26, wherein proteolytically cleavable peptide (CP) consists of the amino acid sequence GMPKDLYHAS (SEQ ID NO:2).
29. The masked cytokine according to any one of clauses 24 to 26, wherein proteolytically cleavable peptide (CP) consists of the amino acid sequence RPLALWRS (SEQ ID NO:3).
30. The masked cytokine according to any one of clauses 24 to 26, wherein proteolytically cleavable peptide (CP) consists of the amino acid sequence TQKPLGLS (SEQ ID NO:4).
31. The masked cytokine according to any one of clauses 24 to 26, wherein proteolytically cleavable peptide (CP) consists of the amino acid sequence APAGLIVPYN (SEQ ID NO:5).
32. The masked cytokine according to any one of clauses 24 to 26, wherein proteolytically cleavable peptide (CP) consists of the amino acid sequence PANLVAPDP (SEQ ID NO:6).
33. The masked cytokine according to any one of clauses 24 to 26, wherein proteolytically cleavable peptide (CP) consists of the amino acid sequence IVGRPRHQGV (SEQ ID NO:7).
34. The masked cytokine according to any one of clauses 24 to 26, wherein proteolytically cleavable peptide (CP) consists of the amino acid sequence RSKYLATA (SEQ ID NO:8).
35. A targeted cytokine comprising:
   a) a targeting moiety;
   b) a cytokine or a fragment thereof;
   c) a masking moiety; and
   d) an Fc domain comprising a first Fc polypeptide linked to the cytokine or a fragment thereof through a first linker and a second Fc polypeptide linked to the masking moiety through a second linker, wherein the first or the second linker is a cleavable linker such that the masking moiety releases the cytokine or a fragment thereof upon cleavage;
      wherein the targeting moiety is linked to the Fc domain through one or both of the first and second Fc polypeptides; and
      wherein at least the first linker or the second linker is a proteolytically cleavable peptide linker comprising a proteolytically cleavable peptide (CP) consisting of the amino acid sequence PVSLRSGS (SEQ ID NO: 1), or GMPKDLYHAS (SEQ ID NO:2), or RPLALWRS (SEQ ID NO:3), or TQKPLGLS (SEQ ID NO:4), or APAGLIVPYN (SEQ ID NO:5), or PANLVAPDP (SEQ ID NO:6), or IVGRPRHQGV (SEQ ID NO:7), or RSKYLATA (SEQ ID NO:8).
36. The targeted cytokine of clause 35, wherein the second linker is the proteolytically cleavable linker and the first linker is a non-cleavable linker.
37. The targeted cytokine of clause 35, wherein the first linker is the proteolytically cleavable linker and the second linker is a non-cleavable linker.
38. The targeted cytokine according to any one of clauses 35 to 37, wherein proteolytically cleavable peptide (CP) consists of the amino acid sequence PVSLRSGS (SEQ ID NO:1).
39. The targeted cytokine according to any one of clauses 35 to 37, wherein proteolytically cleavable peptide (CP) consists of the amino acid sequence GMPKDLYHAS (SEQ ID NO:2).
40. The targeted cytokine according to any one of clauses 35 to 37, wherein proteolytically cleavable peptide (CP) consists of the amino acid sequence RPLALWRS (SEQ ID NO:3).
41. The targeted cytokine according to any one of clauses 35 to 37, wherein proteolytically cleavable peptide (CP) consists of the amino acid sequence TQKPLGLS (SEQ ID NO:4).
42. The targeted cytokine according to any one of clauses 35 to 37, wherein proteolytically cleavable peptide (CP) consists of the amino acid sequence APAGLIVPYN (SEQ ID NO:5).
43. The targeted cytokine according to any one of clauses 35 to 37, wherein proteolytically cleavable peptide (CP) consists of the amino acid sequence PANLVAPDP (SEQ ID NO:6).
44. The targeted cytokine according to any one of clauses 35 to 37, wherein proteolytically cleavable peptide (CP) consists of the amino acid sequence IVGRPRHQGV (SEQ ID NO:7).
45. The targeted cytokine according to any one of clauses 35 to 37, wherein proteolytically cleavable peptide (CP) consists of the amino acid sequence RSKYLATA (SEQ ID NO:8).
46. The targeted cytokine according to any one of clauses 35-45, wherein the targeting moiety is an antibody or an antigen binding domain thereof.
47. A cleavage product preparable by proteolytic cleavage of the proteolytically cleavable linker in a polypeptide or polypeptide construct according to any one of clauses 1 to 22, a cytokine prodrug according to clause 23, or a masked cytokine according to any one of clauses 24 to 34, or a targeted cytokine according to any one of clauses 35 to 46.
48. A nucleic acid encoding a polypeptide or polypeptide construct according to any one of clauses 1 to 22.
49. A vector comprising a nucleic acid according to clause 48.
50. A host cell comprising a nucleic acid according to clause 48, or a vector according to clause 49.
51. A composition comprising the polypeptide or polypeptide construct according to any one of clauses 1 to 22, or cytokine prodrug according to clause 23, or masked cytokine according to any one of clauses 24 to 34, or targeted cytokine according to any one of clauses 35 to 46, or cleavage product according to clause 47.
52. A pharmaceutical composition comprising the polypeptide construct according to any one of clauses 1 to 22, or cytokine prodrug according to clause 23, or masked cytokine according to clauses 24 to 34, or targeted cytokine according to any one of clauses 35 to 46, or cleavage product according to clause 47, and a pharmaceutically acceptable carrier.
53. A kit comprising a polypeptide or polypeptide construct according to any of clauses 1 to 22, or a cytokine prodrug according to clause 23, or a masked cytokine according to any of clauses 24 to 34, or a targeted cytokine according to any one of clauses 35 to 46, or a cleavage product according to clause 47, or a composition according to clause 51, or a pharmaceutical composition according to clause 52.
54. A polypeptide construct according to any one of clauses 13 to 22, or a cytokine prodrug according to clause 23, or a masked cytokine according to any one of clauses 24 to 34, or a targeted cytokine according to any one of clauses 35 to 46, or a cleavage product according to clause 47, or a composition according to clause 51, or a pharmaceutical composition according to clause 52 for use in medicine.
55. A method of treating or preventing cancer in a subject, the method comprising administering to the subject an effective amount of a polypeptide construct according to clauses 13 to 22, or a cytokine prodrug according to clause 23, or a masked cytokine according to any one of clauses 24 to 34, or a targeted cytokine according to any one of clauses 35 to 46, or a cleavage product according to clause 47, or a composition according to clause 51, or a pharmaceutical composition according to clause 52.
56. A polypeptide construct according to any one of clauses 13 to 22, or a cytokine prodrug according to clause 23, or a masked cytokine according to any one of clauses 24 to 34, or a targeted cytokine according to any one of clauses 35 to 46, or a cleavage product according to clause 47, or a composition according to clause 51, or a pharmaceutical composition according to clause 52 for use in treating or preventing cancer.
57. A cleavage product as defined in clause 47 for use in a method of treating or preventing cancer, the method comprising administering a polypeptide drug construct according to any one of clauses 13 to 22, a cytokine prodrug according to clause 23, or a masked cytokine according to any one of clauses 24 to 34, or a targeted cytokine according to any one of clauses 35 to 46, to a patient, thereby producing the cleavage product by proteolytic cleavage of the proteolytically cleavable peptide linker in vivo.

## Claims

1. A masked cytokine comprising:
a) a first polypeptide chain comprising a masking moiety linked to a first half-life extension moiety via a first linker; and
b) a second polypeptide chain comprising a cytokine moiety linked to a second half-life extension moiety via a second linker,
wherein the first half-life extension moiety is associated with the second half-life extension moiety, and
wherein at least the first linker or the second linker is a proteolytically cleavable peptide linker comprising a proteolytically cleavable peptide (CP) consisting of the amino acid sequence GMPKDLYHAS (SEQ ID NO: 2), or RPLALWRS (SEQ ID NO: 3), or TQKPLGLS (SEQ ID NO: 4), or APAGLIVPYN (SEQ ID NO: 5), or PANLVAPDP (SEQ ID NO: 6), or IVGRPRHQGV (SEQ ID NO: 7), or RSKYLATA (SEQ ID NO: 8).

2. The masked cytokine according to claim 1, wherein the second linker is the proteolytically cleavable linker and the first linker is a non-cleavable linker.

3. The masked cytokine according to claim 1, wherein the first linker is the proteolytically cleavable linker and the second linker is a non-cleavable linker.

4. A targeted cytokine comprising:
a) a targeting moiety;
b) a cytokine moiety;
c) a masking moiety; and
d) an Fc domain comprising a first Fc polypeptide linked to the cytokine moiety through a first linker and a second Fc polypeptide linked to the masking moiety through a second linker, wherein the first or the second linker is a cleavable linker such that the masking moiety releases the cytokine moiety upon cleavage;
wherein the targeting moiety is linked to the Fc domain through one or both of the first and second Fc polypeptides; and
wherein at least the first linker or the second linker is a proteolytically cleavable peptide linker comprising a proteolytically cleavable peptide (CP) consisting of the amino acid sequence GMPKDLYHAS (SEQ ID NO: 2), or RPLALWRS (SEQ ID NO: 3), or TQKPLGLS (SEQ ID NO: 4), or APAGLIVPYN (SEQ ID NO: 5), or PANLVAPDP (SEQ ID NO: 6), or IVGRPRHQGV (SEQ ID NO: 7), or RSKYLATA (SEQ ID NO: 8).

5. The targeted cytokine of claim 4, wherein the second linker is the proteolytically cleavable linker and the first linker is a non-cleavable linker.

6. The targeted cytokine of claim 4, wherein the first linker is the proteolytically cleavable linker and the second linker is a non-cleavable linker.

7. The targeted cytokine according to any one of claims 4 to 6, wherein the targeting moiety is an antibody or an antigen binding domain thereof.

8. A nucleic acid encoding the masked cytokine according to any one of claims 1 to 3, or the targeted cytokine according to any one of claims 4 to 7.

9. A vector comprising a nucleic acid according to claim 8.

10. A host cell comprising a nucleic acid according to claim 8, or a vector according to claim 9.

11. A composition comprising the masked cytokine according to any one of claims 1 to 3, or the targeted cytokine according to any one of claims 4 to 7.

12. A pharmaceutical composition comprising the masked cytokine according to claims 1 to 3, or the targeted cytokine according to any one of claims 4 to 7, and a pharmaceutically acceptable carrier.

13. A kit comprising the masked cytokine according to any of claims 1 to 3, or the targeted cytokine according to any one of claims 4 to 7, or the composition according to claim 11, or the pharmaceutical composition according to claim 12.

14. A masked cytokine according to any one of claims 1 to 3, or a targeted cytokine according to any one of claims 4 to 7, or a composition according to claim 11, or a pharmaceutical composition according to claim 12 for use in medicine.

15. A masked cytokine according to any one of claims 1 to 3, or a targeted cytokine according to any one of claims 4 to 7, or a composition according to claim 11, or a pharmaceutical composition according to claim 12 for use in treating or preventing cancer.
